# EUROPÄISCHE PATENTSCHRIFT

(11) **EP 0 707 591 B1**
(45) Veröffentlichungstag und Bekanntmachung des Hinweises auf die Patenterteilung: **26.09.2001**
(21) Anmeldenummer: 94919670.3
(22) Anmeldetag: 29.06.1994
(51) Int. Cl.: C07H 21/00, A61K 31/70

(54) **METHYLPHOSPHONSÄUREESTER, VERFAHREN ZU DEREN HERSTELLUNG UND DEREN VERWENDUNG**
METHYLPHOSPHONIC ACID ESTER, PROCESS FOR PREPARING THE SAME AND ITS USE
ESTERS D'ACIDE METHYLPHOSPHONIQUE, LEUR PROCEDE DE PREPARATION ET LEUR UTILISATION

(30) Priorität: 01.07.1993 DE 4321946
(43) Veröffentlichungstag der Anmeldung: 24.04.1996
(73) Patentinhaber: Aventis Pharma Deutschland GmbH, 65929 Frankfurt am Main (DE)
(72) Erfinder: UHLMANN, Eugen, D-61479 Glashütten (DE); MEIER, Chris, D-61352 Bad Homburg (DE)
(86) Internationale Anmeldenummer: EP9402121
(87) Internationale Veröffentlichungsnummer: WO9501363

(56) Entgegenhaltungen:
- WO-A-93/10140
- CHEMICAL ABSTRACTS, vol. 120, no. 25, 20. Juni 1994, Columbus, Ohio, US; abstract no. 324093k, MEIER, C. '5',5'-O-Dinucleoside (alpha-hydroxybenzyl)phosphonates as a lipophilic potential prodrug of ddT' Seite 974 ; & ANGEW. CHEMIE Bd. 105, Nr. 12 , 1993 Seiten 1854 - 6
- TETRAHEDRON LETT. Bd. 33 , 1992 Seiten 2357 - 60 ROELEN, H. 'Synthesis of Alkylphosphon(othio)ate Analogues of DNA'
- BIOORG. MED. CHEM. LETT. Bd. 1 , 1991 Seiten 527 - 30 MEIER, C. 'O-Alkyl-5',5'-dinucleoside-phosphates as combined prodrugs of antiviral and antibiotic compounds'

## Beschreibung

Gegenstand der vorliegenden Erfindung sind Methylphosphonsäureester, Verfahren zu deren Herstellung und deren Verwendung.

Es sind bereits phosphorylierte Wirkstoffderivate bekannt, die teilweise auch zu pharmazeutischen Zwecken eingesetzt wurden. So werden z.B. in J. Med. Chem. 1993, 36, 1048-1052 Phosphoamidatester mit AZT beschrieben. Die anitvirale Aktivität dieser Verbindungen ist jedoch um den Faktor 10 kleiner als die von AZT und die Toxizität der genannten Verbindungen ist um den Faktor 5 höher als die von AZT. In J. Med. Chem. 1991, 34, 1830-1837 werden Phosphotriester-Derivate mit AZT beschrieben; auch bei diesen Verbindungen ist die Aktivität geringer und die Toxizität größer als bei AZT. Ähnliche Ergebnisse werden mit verwandten Verbindungen erzielt, über die in J. Org. Chem. 1992, 57, 7300-7307 berichtet wird.

Die Literaturstelle Tetrahedron Lett. vol. 33, p. 2357-60 schlägt alkylphosphonat-verbrückte Oligonukleotide für Antisensstudien vor. Der Patentanmeldung WO-A-93 10 140 kann ferner entnommen werden, daß die Phosphonalkylgruppe solcher Oligonukleotide funktionalisiert werden kann, und zwar durch Einführung ionischer Gruppen in die Alkylkette. Die Literaturstelle Bioorg. Med. Chem. Lett. vol 1, 1991, p.527-30 schließlich belegt, daß lipophile Dinukleotide bestehend aus bewährten anti-HIV-Mononukleotiden (AZT usw.) als Mittel gegen HIV interessant sein können.

In der Absicht, phosphorylierte Wirkstoffderivate zu erhalten, die nicht die Nachteile einschlägiger Verbindungen des Standes der Technik aufweisen, wurde nun gefunden, daß die erfindungsgemäßen Methylphosphonsäureester hervorragende Eigenschaften haben. Erfindungsgegenstand sind demzufolge
1) Verbindungen der Formel I, 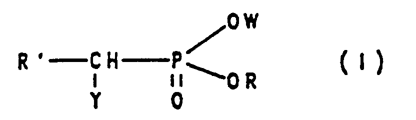 dadurch gekennzeichnet, daß
   Y die Bedeutung von OH, SH, OAc oder SAc, mit Ac=(C1-C18)-Acyl, ggf. 1-3-fach ungesättigt, hat,
   R' Aryl, Heteroaryl oder Alkyl bedeutet,
   W die Bedeutung eines pharmazeutisch wirksamen 5', 3' oder 2' Nukleosid-Analogons hat,
   R die Bedeutung von W hat wobei R und W gleich oder verschieden sein können, oder R bedeutet einen ggf. substituierten Alkylrest oder
   W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet 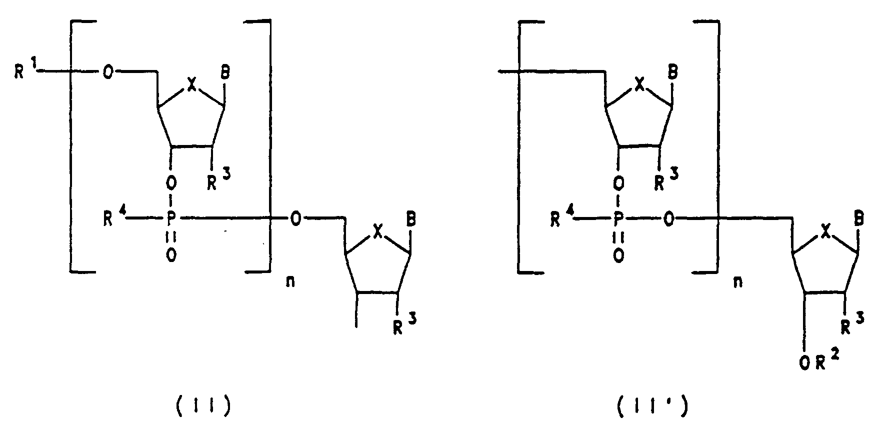 wobei X Oxy, Sulfandiyl oder Methylen bedeutet,
   B unabhängig voneinander eine Nukleotidbase darstellt,
   n unabhängig voneinander eine ganze Zahl von 0 bis 50 bedeutet,
   R¹ und R² bedeuten unabhängig voneinander H (C1-C18)-Acyl oder einen Rest der Formel 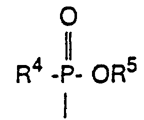
   worin R⁴ O⁻, S⁻, CH₃ oder CHYR', mit R' und Y wie oben definiert bedeutet und R⁵ einen gegebenenfalls substituierten Alkylrest mit 1-18 C-Atomen bedeutet,
   R³ bedeutet unabhängig voneinander H, O(C1-C18)-Alkyl, O(C1-C18)-Acyl, F, Cl, N₃, NH₂ oder NHR⁶ wobei R⁶ (C1-C6)-Alkyl oder -Acyl
   und die geschweifte Klammer deutet an, daß R³ und der benachbarte Phosphonylrest sich in 2' bzw. 3' Position befinden können.
2) Bevorzugt sind Verbindungen der Formel I wie unter 1) erläutert,
   dadurch gekennzeichnet, daß
   Y die Bedeutung von OH, SH, OAc oder SAc, mit Ac=(C1-C8)-Acyl, ggf. 1-3-fach ungesättigt, hat,
   R' Aryl mit 6-14 C-Atomen, ggf. substituiert mit bis zu drei voneinander unabhängigen Resten ausgewählt aus der Gruppe (C1-C5)-Alkyl, Halogen, NO₂, CN, (C1-C6)-Alkoxy, Amino, (C1-C4)-Alkylamino, (C1-C8)-Dialkylamino, wobei an den Arylrest auch ein (C3-C8)-Alkylenrest ankondensiert sein kann, in dem eine CH₂-Gruppe auch durch Oxy ersetzt sein kann;
   Heteroaryl mit 3 bis 13 C-Atomen und bis zu drei Heteroatomen ausgewählt aus der Gruppe N, O und S;
   (C1-C16)-Alkyl, verzweigt oder unverzweigt, gesättigt oder 1-3 fach ungesättigt, ggf. substituiert unabhängig voneinander mit bis zu drei Substituenten ausgewählt aus der Gruppe Halogen, CN, NO₂ und (C1-C3)-Alkoxy bedeutet,
   W die Bedeutung eines pharmazeutisch wirksamen 5', 3' oder 2' Nukleosid-Analogons hat,
   R die Bedeutung von W hat, wobei R und W gleich oder verschieden sein können, oder R bedeutet oder (C1-C16)-Alkyl, das verzweigt oder unverzweigt sein kann und ggf. substituiert ist unabhängig voneinander mit bis zu 3 Resten aus der Gruppe Halogen, CN, (C1-C8)-Acyloxy, (C1-C18)-Alkoxy oder
   W und R bilden zusammen mit dem sie tragenden Phosphonat-Rest ein Oligonukleotid wobei W einen Rest der Formel II, oder ein modifiziertes Oligonukleotid und R einen Rest der Formel II' oder ein modifiziertes Oligonukleotid bedeutet
   wobei X Oxy oder Sulfandiyl bedeutet,
   B unabhängig voneinander eine Nukleotidbase darstellt,
   n unabhängig voneinander eine ganze Zahl von 0 bis 30 bedeutet,
   R¹ und R² bedeuten unabhängig voneinander H (C1-C12)-Acyl oder einen Rest der Formel 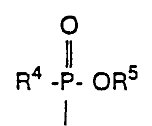
   worin R⁴ O, S, CH₃ oder CHYR', mit R' und Y wie oben definiert bedeutet und R⁵ einen gegebenenfalls substituierten Alkylrest mit 1-12 C-Atomen bedeutet,
   R³ bedeutet unabhängig voneinander H, O(C1-C12)-Alkyl, O(C1-C12)-Acyl, Cl, N₃, NH₂ oder NHR⁶ wobei R⁶ (C1-C3)-Alkyl oder -Acyl
   und die geschweifte Klammer deutet an, daß R³ und der benachbarte Phosphonylrest sich in 2' bzw. 3' Position befinden können,
3) Besonders bevorzugt sind Verbindungen der Formel I wie unter 1) oder 2) erläutert,
   dadurch gekennzeichnet, daß
   Y die Bedeutung von OH, SH, OAc oder SAc hat, mit Ac=(C1-C3)-Acyl, ggf. 1-3-fach ungesättigt,
   R' Aryl mit 6-14 C-Atomen, ggf. substituiert mit bis zu 3 voneinander unabhängigen Resten ausgewählt aus der Gruppe (C1-C3)-Alkyl, F, Cl, NO₂, CN, (C1-C4)-Alkoxy, Amino, (C1-C3)-Alkylamino, (C1-C6)-Dialkylamino, wobei an den Arylrest auch ein (C3-C8)-Alkylenrest ankondensiert sein kann, in dem eine CH₂-Gruppe auch durch Oxy ersetzt sein kann;
   Heteroaryl mit 3 bis 6 C-Atomen und bis zu drei Heteroatomen ausgewählt aus der Gruppe N, O und S;
   (C1-C8)-Alkyl, verzweigt oder unverzweigt, gesättigt oder 1-3 fach ungesättigt, ggf. substituiert unabhängig voneinander mit bis zu drei Substituenten ausgewählt aus der Gruppe Cl, CN, NO₂ und (C1-C3)-Alkoxy bedeutet,
   W die Bedeutung eines pharmazeutisch wirksamen 5'-, 3' oder 2' Nukleosid-Analogons hat,
   R die Bedeutung von W hat oder (C1-C8)-Alkyl bedeutet, das verzweigt oder unverzweigt sein kann und ggf. substituiert ist mit bis zu 2 Resten aus der Gruppe Halogen, CN, (C3-C6)-Acyloxy, (C8-C18)-Alkoxy oder
   W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet
   wobei X Oxy bedeutet,
   B unabhängig voneinander eine Nukleotidbase darstellt,
   n unabhängig voneinander eine ganze Zahl von 0 bis 20 bedeutet,
   R¹ und R² bedeuten unabhängig voneinander H (C1-C8)-Acyl oder einen Rest der Formel 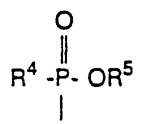
   worin R⁴ O, S, CH₃ oder CHYR', mit R' und Y wie oben definiert bedeutet und R⁵ einen gegebenenfalls substituierten Alkylrest mit 1-8 C-Atomen bedeutet,
   R³ bedeutet unabhängig voneinander H, O(C1-C8)-Alkyl, O(C1-C8)-Acyl, Cl oder N₃, und die geschweifte Klammer deutet an, daß R³ und der benachbarte Phosphonylrest sich in 2' bzw. 3' Position befinden können.
4) Ganz besonders bevorzugt sind Verbindungen der Formel I wie oben unter 1) bis 3) erläutert,
   dadurch gekennzeichnet, daß
   Y die Bedeutung von OH hat,
   R' Aryl mit 6 C-Atomen, ggf. substituiert mit bis zu 3 voneinander unabhängigen Resten ausgewählt aus der Gruppe
   (C1-C3)-Alkyl, F, Cl, NO₂, CN, (C1-C4)-Alkoxy, Amino, (C1-C3)-Alkylamino, (C1-C6)-Dialkylamino, wobei an den Arylrest auch ein (C3-C6)-Alkylenrest ankondensiert sein kann, in dem eine CH₂-Gruppe auch durch Oxy ersetzt sein kann;
   Heteroaryl mit 3 bis 6 C-Atomen und bis zu drei Heteroatomen ausgewählt aus der Gruppe N, O und S;
   (C1-C8)-Alkyl, verzweigt oder unverzweigt, gesättigt oder 1-3 fach ungesättigt, vorzugsweise ungesättigt in konjugierter Form mit einer ungesättigten Bindung in alpha-Stellung, ggf. substituiert unabhängig voneinander mit bis zu drei Substituenten ausgewählt aus der Gruppe Cl, CN, NO₂ und (C1-C3)-Alkoxy bedeutet,
   W die Bedeutung eines pharmazeutisch wirksamen 5'- oder 3' Nukleosid-Analogons hat,
   R die Bedeutung von W hat oder (C1-C4)-Alkyl bedeutet, das verzweigt oder unverzweigt sein kann oder
   W und R bilden zusammen mit dem sie tragenden Phosphonat-Rest ein Oligonukleotid wobei W einen Rest der Formel II, oder ein modifiziertes Oligonukleotid und R einen Rest der Formel II' oder ein modifiziertes Oligonukleotid bedeutet
   wobei X Oxy bedeutet,
   B unabhängig voneinander eine Nukleotidbase darstellt,
   n unabhängig voneinander eine ganze Zahl von 0 bis 15 bedeutet,
   R¹ und R² bedeuten unabhängig voneinander H (C1-C4)-Acyl oder einen Rest der Formel 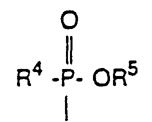
   worin R⁴ O, S, CH₂ oder CHYR', mit R' und Y wie oben definiert bedeutet und R⁵ einen gegebenenfalls substituierten Alkylrest mit 1-3 C-Atomen bedeutet,
   R³ bedeutet unabhängig voneinander H, O(C1-C3)-Alkyl, O(C1-C3)-Acyl, Cl oder N₃, und die geschweifte Klammer deutet an, daß R³ und der benachbarte Phosphonylrest sich in 2' bzw. 3' Position befinden können.
5) Weiterhin von besonderer Bedeutung sind Verbindungen der Formel I wie unter 1) bis 4) erläutert, dadurch gekennzeichnet, daß
   Y die Bedeutung von OH hat,
   W die Bedeutung eines pharmazeutisch wirksamen 5'- oder 3' Nukleosid-Analogons hat,
   R die Bedeutung von W hat oder (C1-C4)-Alkyl bedeutet, das verzweigt oder unverzweigt sein kann,
   R' bedeutet Aryl mit 6 C-Atomen, ggf. substituiert mit bis zu 3 voneinander unabhängigen Resten ausgewählt aus der Gruppe
   Cl, NO₂, CN, (C1-C3)-Alkoxy, Amino und (C1-C3)-Alkylamino.

Der vorstehend im Zusammenhang mit mehrfach auftretenden Substituenten (z.B. "B") gebrauchte Begriff "unabhängig voneinander" soll verdeutlichen, daß in jeweils einer Verbindung die jeweiligen Substituenten unterschiedlich sein können, was auch für sich n-mal wiederholende Elemente gilt.

Beispiele für in den vorangegangenen Definitionen erwähnten Acylgruppen sind Acetyl, Butyryl, Pivaloyl, Crotonoyl, Pentanoyl, Hexanoyl, Octadecanoyl oder Oleyl.

Geeignete Alkylgruppen sind z.B.Methyl, Ethyl, Propyl, Butyl, Isobutyl, Pentyl oder Hexyl.

Beispielhafte Arylgruppen sind Phenyl oder Naphthyl.

Geeignete Heteroarylgruppen sind z. B.Pyridyl, Oxazol, Furyl, Benzofuryl oder Phenothiazinyl.

Beispielhafte Alkylaminogruppen sind die Methyl- und die Dimethylaminogruppen.

Beispielhafte Dialkylaminogruppen sind die Dimethylamino- und die Diethylaminogruppe.

Erfindungsgemäß besonders geeignete pharmazeutisch wirksame Nukleosid-Analoga sind von den Basen Adenin, Cytosin, Guanin, Thymin, Purin, 7-Deazaadenin, 7-Deazaguanin oder 5-Chlorcytosin abgeleitete Verbindungen, insbesondere z.B. 3'-Deoxy-3'-azidothymidin, 2',3'-Dideoxy-2',3'-didehydrothymidin, 2',3'-Dideoxythymidin, 2',3'-Dideoxyuridin, 2',3'-Dideoxyadenosin, 2',3'-Dideoxyinosin, 3'F,3'-Deoxythymidin, Acyclovir und Gancyclovir.

Die obengenannten Reste R⁵ können gegebenenfalls mit Halogen, vorzugweise Cl, CF₃, CN, NH₂ oder (C1-C6)- vorzugsweise (C1-C3)-Alkoxy substiuiert sein.

Zum Gegenstand der vorliegenden Erfindung gehört weiterhin ein Verfahren zur Herstellung von Verbindungen gemäß einem oder mehreren der Ansprüche 1-5 das
dadurch gekennzeichnet, daß
a) eine Verbindung der Formel III mit einer Verbindung der Formel IV umgesetzt wird, 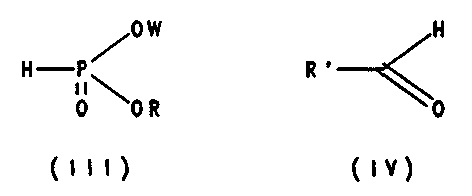 oder daß
b) eine Verbindung der Formel V in beliebiger Reihenfolge und unter Anwendung eines Kondensationsmittels mit Verbindungen der Formel VI umgesetzt wird, oder daß
c) eine Verbindung der Formel V in beliebiger Reihenfolge und unter Anwendung eines Kondensationsmittels mit Verbindungen der Formel VI und der Formel VII umgesetzt wird, 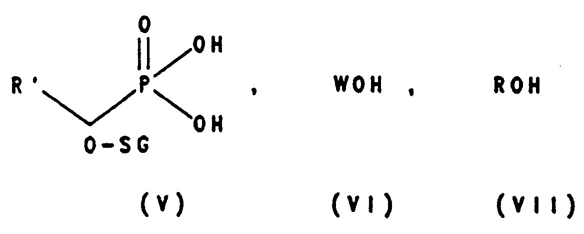
wobei SG eine Schutzgruppe darstellt, die zur Gewinnung der Verbindung der Formel I ggf. abgespalten wird,
oder eine Nucleotideinheit mit 3'(2')-terminaler H-Phosphonat Gruppierung und geschützter 5'-Hydroxygruppe mit einer weiteren Nucleotideinheit mit freier 5'-Hydroxygruppe und geschützter 3'(2')-Hydroxygruppe in Gegenwart eines Aktivierungsmittels zum H-Phosphosphonatdinucleosid umgesetzt und dieses mit einem Aldehyd zum Dinucleosid-α-hydroxyalkyl(aryl)-phosphonat kondensiert wird, das nach Umsetzung zu deren aktivierten Derivaten mit weiteren (Oligo)-Nucleotidfragmenten zu Oligonucleotiden reagiert, wobei temporär eingeführte Schutzgruppen abgespalten werden, oder daß
a) eine Nucleotideinheit mit 3'(2')-terminaler Phosphor(III) oder Phosphor(V)-Gruppierung mit einer freien 5'-Hydroxygruppe einer weiteren Nucleotideinheit bzw. wachsenden Oligonucleotidkette in Gegenwart eines Kondensationsmittels oder
b) deren aktivierten Derivaten umgesetzt wird, oder das Oligonucleotidanalogen in Fragmenten in gleicher Weise aufgebaut wird, in den nach a) oder b) erhaltenen Oligonucleotiden zum Schutz anderer Funktionen temporär eingeführte Schutzgruppen abgespalten werden und die so erhaltenen Oligonucleotid-Analoga der Formel I worin W einen Rest der Formel II oder ein modifiziertes Oligonukleotid und R einen Rest der Formel II' oder ein modifiziertes Oligonukleotid bedeutet gegebenenfalls in ihr physiologisch verträgliches Salz überführt werden.

Die unter a) beschriebene Reaktion läuft vorzugsweise unter folgenden Bedingungen ab.
A) Reaktion des Phosphonatdiesters der Formel III mit den entsprechenden substituierten Aldehyden der Formel IV in einem organischen Lösungsmittel, z.B. in trockenem Triethylamin (NEt₃) bei erhöhter Temperatur, vorzugsweise in der Siedehitze.
B) Reaktion des Phosphonatdiesters der Formel III mit dem Aldehyd der Formel IV in einem trockenen aprotischen Lösungsmittel, z.B. Tetrahydrofuran (THF) unter Zugabe einer organischen Base, z.B. NEt₃ oder Chinin bei Raumtemperatur ±10 °C.

Nach erfolgter Reaktion werden die Produkte nach bekannten Methoden aufgereinigt, z.B. durch Chromatographie.

Die unter b) und c) beschriebenen Reaktionen laufen unter im Stand der Technik für Veresterungen bekannten Bedingungen ab. Besonders gute Ergebnisse erzielt man mittels intermediärer Aktivesterbildung z.B. mit Triazol/Dimesitylensulfonsäurechlorid.
Geeignete Schutzgruppen, die ggf. nach der Reaktion gemäß Methoden des Standes der Technik abgespalten werden, sind z.B. Alkylsilyl, Alkylarylsilyl und Acyl, insbesondere t-Butyldimethylsilyl. Die letztgenannte Schutzgruppe läßt sich vorteilhaft mit Ammoniumfluorid in Methanol abspalten.
Die erfindungsgemäßen Verbindungen lassen sich auch stereoselektiv nach verschiedenen Methoden des Standes der Technik herstellen. Ein bevorzugter Ansatz zur Einführung der Chiralität am α-Kohlenstoff-Atom ist die Reaktion des C-Anions eines t-Butyldimethylsilyl-geschützten Alkohols (Verbindung der Formel V) mit dem aus (+)-Ephedrin als chiralem Auxiliar abgeleitetem Oxazaphospholidin. Das Oxazaphospholidin wird z.B. durch Reaktion von Phosphorylchlorid mit (+)-Ephedrin in 60 %iger Ausbeute und einem Diastereomerenverhältnis von 24:1 erhalten. Eine weitere Möglichkeit zur Einführung der Chiralität besteht in der enantioselektiven Oxazaborolidin-katalysierten Reduktion (Tetrahedron Lett., 31, 611, (1990).

Die zur Durchführung der obengenannten Reaktionen benötigten Ausgangsstoffe sind käuflich, oder können gemäß allgemein bekannter Vorschriften hergestellt werden. Einige bevorzugte Herstellungsmethoden werden in den Beispielen beschrieben.
Die Nucleosid-H-Phosphonatdiester der Formel III, die als Ausgangsverbindungen dienen, lassen sich z.B. durch die Umsetzung von Diisopropylamindichlorphosphin mit den entsprechenden Nucleosiden zum Phosphoramidit, das direkt unter Tetrazol Aktivierung mit Wasser in einer "Eintopf-Reaktion" zu den Verbindungen der Formel III hydrolysiert werden kann, herstellen.

Alternativ gelingt die Synthese z.B. durch die Veresterung eines 5'-Nucleosidphosphorigsäuremonoesters mit einem zweiten Äquivalent des Nucleosids unter Pivaloylchlorid-Aktivierung. Ein 5'-Nucleosid-phosphorigsäuremonoester ist durch Reaktion von Phosphortrichlorid mit Imidazol zum Phosphortriimidazolid, nach Reaktion mit dem entsprechenden Nucleosid und anschließender Hydrolyse zugänglich.

Alternativ lassen sich die Verbindungen der Formel III durch die Veresterung eines 5'-Nucleosid-phosphorigsäuremonoesters mit einem zweiten Äquivalent des entsprechenden Nucleosids unter Pivaloylchlorid-Aktivierung herstellen.

Die Herstellung der Verbindungen der Formel I mit einem Rest der Formel II und II' erfolgt vorzugsweise so, daß man im Prinzip wie oben beschrieben dimere Nucleotide der Formel XI herstellt, die dann nach gängigen Methoden in Oligonucleotide eingebaut werden. Beispielsweise (Schema 1) kann ein 5'-geschützter-Nucleosid-3'-H-phosphonatester der Formel VIII mit einer 3'-geschützten 5'-Hydroxy-Komponente der Formel IX in Gegenwart eines Kondensationsmittels wie Pivaloylchlorid in Pyridin zum Dinucleosid H-Phosphonatester der Formel X umgesetzt werden. Dieser wird anschließend mit dem entsprechenden Aldehyd zum Dinucleosid hydroxyalkylphosphonat umgesetzt. Die freie α-Hydroxygruppe muß für weitere Umsetzungen geschützt werden, vorzugsweise mit der TBDMS (t-Butyldimethylsilyl)-Schutzgruppe, die am Ende der Synthese mit Fluoridionen abgespalten wird. Formel XI Verbindungen werden beispielsweise nach Abspaltung der 3'-Schutzgruppe (SG²) zum Phosphoramidit der Formel XII umgesetzt, die sich als Analoge der Phosphoramidite in Oligonucleotide nach bekannten Methoden einführen lassen.
Die Pro-Drug Nukleotide lassen sich aber auch als monomere Einheiten durch Kondensation entsprechend geschützter Nucleosid-3' (bzw. 5')-phosphonatester mit der 5' (bzw. 3'-)-Hydroxygruppe eines 3' (bzw. 5'-) geschützten Nucleosids aufbauen (Schema 2). Vorzugsweise verwendet man die Nucleosid-3'-phosphonamidate der Formel XIII (wobei (P) = P-N(ipropyl)₂ ist), die sich nach gängigen Methoden in Oligonucleotide einbauen lassen.

Die Darstellung von Oligonucleotidanaloga der Formel I mit einem Rest der Formel II oder II' erfolgt ähnlich wie die Synthese biologischer Oligonucleotide in Lösung oder vorzugsweise an fester Phase, gegebenenfalls unter Zuhilfenahme eines automatischen Synthesegeräts.

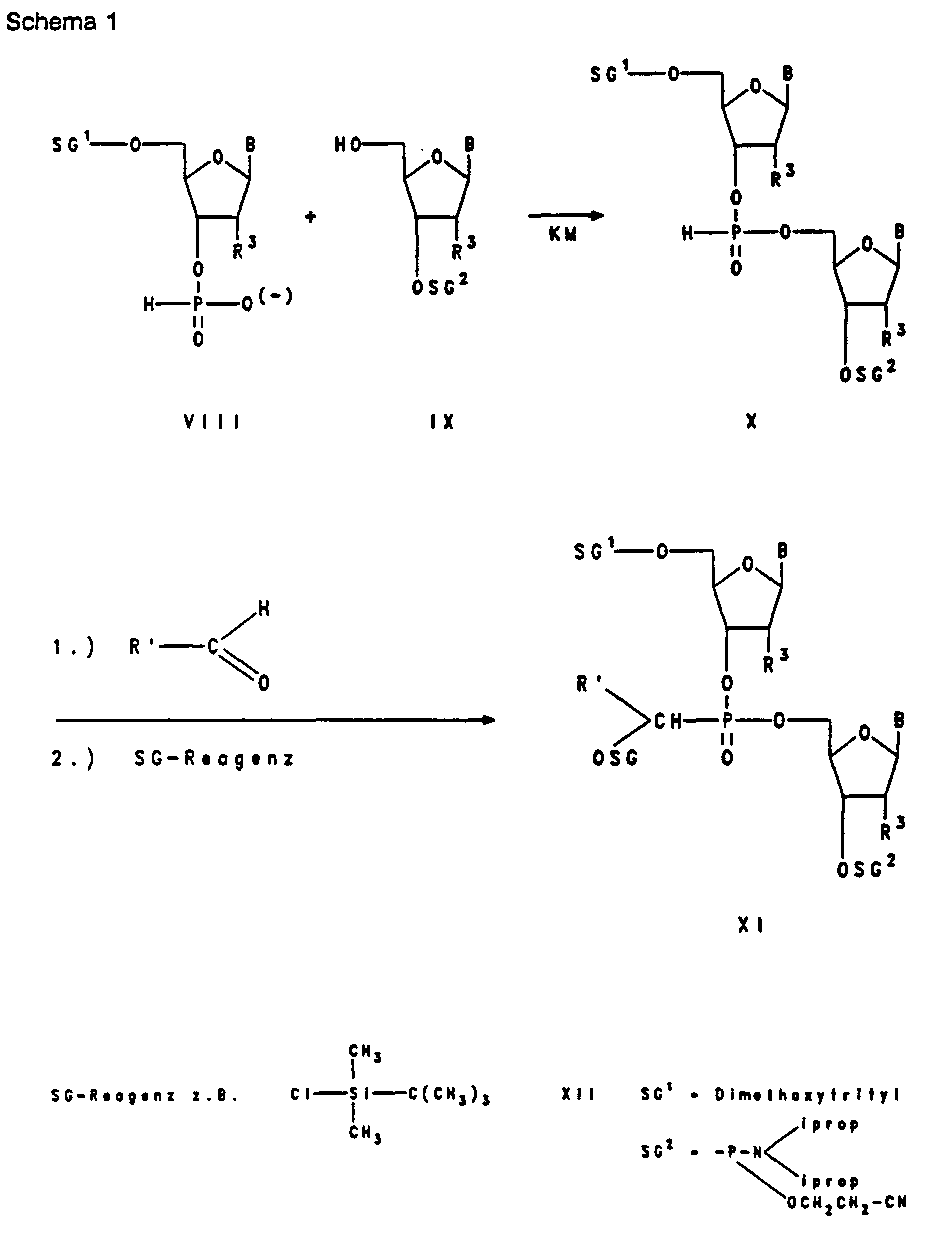

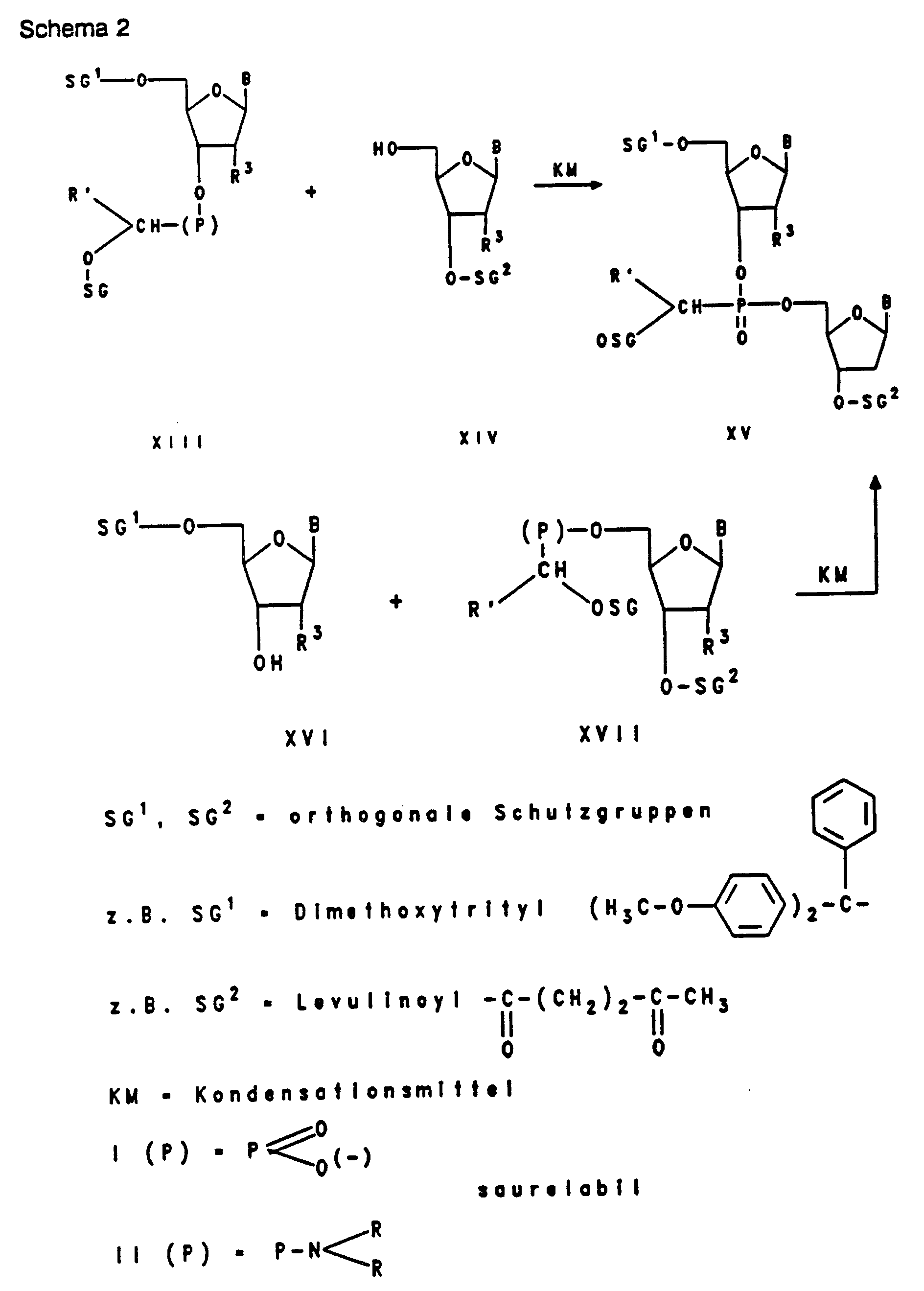

Die erfindungsgemäßen Verbindungen der Formel I und ihre pharmazeutisch verträglichen Salze zeigen die pharmazeutische Wirksamkeit der zugrundeliegenden Wirkstoffe. Da sie zudem günstige toxikologische und pharmakokinetische Eigenschaften zeigen, stellen sie wertvolle Chemotherapeutika dar.

Die Erfindung betrifft somit auch Arzneimittel, insbesondere Arzneimittel zur Bekämpfung von Viruserkrankungen, die durch einen Gehalt an einer oder mehreren der erfindungsgemäßen Verbindungen charakterisiert sind. Sie können z.B. oral, intramuskulär oder intravenös verabfolgt werden.

Arzneimittel, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmittel, wie z. B. Puffersubstanzen vermischt, und in eine geeignete Zubereitungsform bringt.

Als Verdünnungsmittel seien beispielsweise erwähnt Polyglykole, Ethanol und Wasser. Puffersubstanzen sind beispielsweise organische Verbindungen, wie z.B. N', N'-Dibenzylethylendiamin, Diethanolamin, Ethylendiamin, N-Methylglucamin, N-Benzylphenethylamin, Diethylamin, Tris(hydroxymethyl)aminomethan, oder anorganische Verbindungen, wie z. B. Phosphatpuffer, Natriumbicarbonat, Natriumcarbonat. Für die orale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der Formel I oder ihrer pharmazeutisch verträglichen Salze sind stark abhängig von den jeweiligen zugrundeliegenden Wirkstoffen; z.B. im Falle des AZT liegen sie bei etwa 0,4 g vorzugsweise 0,5 g bis maximal 20 g pro Tag für einen Erwachsenen von etwa 75 kg Körpergewicht. Es können Einzel- oder im allgemeinen Mehrfachdosen verabreicht werden, wobei die Einzeldosis den Wirkstoff in einer Menge von etwa 50 is 1000 mg enthalten kann.

Die vorliegende Erfindung betrifft weiterhin die Verwendung der neuen Oligonucleotidanaloga (Verbindungen der Formel I mit einem Rest der Formel II oder einem modifizierten Oligonukleotid und verbindungen der Formel II' oder einem modifizierten Oligonukleotid zur Herstellung eines Arzneimittels (Antisense Oligonucleotide, Ribozyme, Sense Oligonucleotide und Triplex Forming Oligonucleotide).
Oligonucleotide finden in wachsendem Maße Anwendung als Inhibitoren der Genexpression (G. Zon, Pharmaceutical Research 5, 539 (1988); J. S. Cohen, Topics in Molecular and Structural Biology 12 (1989) Macmillan Press; C. Helene and J. J. Toulme, Biochemica et Biophysica Acta 1049, 99 (1990); E. Uhlmann and A. Peyman, Chemical Reviews 90, 543 (1990)). Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Enzymen, Immunmodulatoren, lonenkanälen oder Onkogenen in Frage. Die Inhibition der Virus Vermehrung mit Hilfe von Antisense Oligonucleotiden wurde beispielsweise für RSV (Rous Sarcoma Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind. Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (Helene, 1990). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus. Während mit Hilfe der Antisense Oligonucleotide im allgemeinen die Prozessierung (Splicing etc.) der mRNA oder deren Translation in das Protein gehemmt werden, hemmen Triplex Forming Oligonucleotide die Transkription oder Replikation der DNA (Helene et al., 1990, Uhlmann und Peyman, 1990). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein. Eine weitere Anwendung synthetischer Oligonucleotide sind die sogenannten Ribozyme, welche die Target-RNA infolge ihrer Ribonuclease-Aktivität zerstören (J.J. Rossi and N. Sarver, TIBTECH 8, 179 (1990).

Für die meisten genannten Anwendungen sind Oligonucleotide in ihrer natürlich vorkommenden Form wenig oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:
1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen-spezifischer Weise an ihre Target-Nucleinsäure binden, um den inhibitorischen Effekt zu entfalten.

Wenn die Internucleotid-Phosphatreste permanent verändert werden, verändern sich die Eigenschaften der Oligonucleotide oft drastisch. Beispielsweise wirken Phosphorothioat-Oligonucleotide oft sequenzunspezifisch.

Weitere Aufgabe der Erfindung ist daher, Oligonucleotidanaloge mit spezifischer Wirksamkeit und erhöhter Serumstabilität bereitzustellen, die sich in biologischen Systemen (Serum, Organ, Zelle) wieder in ihre natürlichen Phosphodiester Oligonucleotide zurückverwandeln.

Es können ein oder mehrere Internucleotid-Phosphatreste in den Oligonucleotiden als Pro-Drug modifiziert werden. Es wurde gefunden, daß Oligonucleotide mit 3' und/oder 5'-terminaler Pro-Drug Modifikation bereits im Serum stabiler sind als die natürlich vorkommenden Phosphodiester-Oligonucleotide.

Die Erfindung ist nicht auf α- und β-D- bzw. L-Ribofuranoside, α- und β-D- bzw. L-Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise ringerweiterte und ringverengte Zucker, acyclische, ringverbrückte oder geeignete andersartige Zucker-Derivate. Die Erfindung ist ferner nicht auf die in Formel I beispielhaft aufgeführten Derivate des Phosphat-Restes beschränkt, sondern bezieht sich auch auf die bekannten Dephospho-Derivate.

Die Oligonucleotide können also in vielfältiger Weise von der natürlichen Struktur abgewandelt sein. Solche Modifikationen, die nach an sich bekannten Methoden eingefürt werden, sind beispielsweise:
a) Modifikationen der Phosphatbrücke
   Beispielhaft seien genannt: Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate, Phosphorodithioate und Methylphosphonate.
b) Ersatz der Phosphatbrücke
   Beispielhaft seien genannt: Ersatz durch Formacetal, 3'-Thioformacetal, Methylhydroxylamin, Oxim, Methylendimethylhydrazo, Dimethylensulfon, Silylgruppen. Bevorzugte ist der Ersatz durch Formacetale und 3'-Thioformacetale.
c) Modifikationen des Zuckers
   Beispielhaft seien genannt: α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, Carbocyclische Zuckeranaloge. Bevorzugte Modifikation ist die durch 2'-O-Methylribose und 2'-O-n-Butylribose.
d) Modifikationen der Basen welche die Spezifität der Watson-Crick Basenpaarung nicht verändern
   Beispielhaft seien genannt: 5-Propinyl-2'-desoxyuridin, 5-Propinyl-2'-desoxycytidin, 5-Hexinyl-2'-desoxyuridin, 5-Hexinyl-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin. Bevorzugte Modifikationen sind 5-Propinyl-2'-desoxyuridin, 5-Hexinyl-2'-desoxyuridin, 5-Hexinyl-2'-desoxycytidin und 5-Propinyl-2'-desoxycytidin.
e) 3'-3'- und 5'-5'-Inversionen [z.B. M. Koga et al., J. Org. Chem. 56 (1991) 3757]
f) 5'- und 3'-Phosphate, sowie 5'- und 3'-Thiophosphate.

Beispielhaft für Gruppen, die die intrazelluläre Aufnahme begünstigen, sind verschiedene lipophile Reste wie -O-(CH₂)ₓ-CH₃, worin x eine ganze Zahl von 6 bis 18 bedeutet, -O-(CH₂)ₙ-CH=CH-(CH₂)ₘ-CH₃, worin n und m unabhängig voneinander eine ganze Zahl von 6 bis 12 bedeuten, -O-(CH₂CH₂O)₄-(CH₂)₉-CH₃,-O-(CH₂CH₂O)₈₋ (CH₂)₁₃-CH₃ und -O-(CH₂CH₂O)₇-(CH₂)₁₅-CH₃, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor).

Der Aufbau des Oligonucleotids erfolgt nach den dem Fachmann bekannten Verfahren wie der Triester-Methode, der H-Phosphonat-Methode oder Phosphoramidit-Methode, bevorzugt nach der Standard-Phosphoramidit Chemie nach Caruthers (M. D. Matteucci and M. H. Caruthers, J. Am. Chem. Soc. 103, 3185 (1981)).

Es wurde ferner gefunden, daß Verbindungen der Formel I, in denen W gleich Formel II und R gleich Formel II' bedeuten, in Abhängigkeit von der Basenfolge des DNA-Teils die Expression spezifischer Gene, beispielsweise von Enzymen, Rezeptoren oder Wachstumsfaktoren in Zellkultur und in ausgewählten Beispielen im Tiermodell hemmen.

Ganz generell erstreckt sich die vorliegende Erfindung auf die Verwendung von Verbindungen der Formel I als therapeutisch wirksame Bestandteile eines Arzneimittels. Als therapeutisch wirksame Oligonucleotid-Derivate versteht man im allgemeinen Antisense Oligonucleotide, Tripelhelix-bildende-Oligonucleotide, Aptamere oder Ribozyme, insbesondere Antisense-Oligonucleotide.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Antisense Oligonucleotid-Derivate, die gegen solche Targets wirksam sind, haben beispielsweise folgende Basensequenz:
a) gegen HIV, z. B. 
5'-A C A C C C A A T T C T G A A A A T G G -3' oder
(I)
 
5′-A G G T C C C T G T T C G G G C G C C A-3′ oder
(II)
 
5'-G T C G A C A C C C A A T T C T G A A A A T G G A T A A-3' oder
(III)
 
5′-G C T A T G T C G A C A C C C A A T T C T G A A A-3′ oder
(IV)
 
5′-T C G T C G C T G T C T C C G C T T C T T C T T C C T G C C A oder
(VI)
b) gegen HSV-1, z.B.

5'-G C G G G G C T C C A T G G G G G T C G-3'
(VII)

Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl
3) Zelluläre Rezeptoren wie beispielsweise EGF-Rezeptor, c-erbA, Retinoid-Rezeptoren, Protein-Kinase regulatorische Untereinheit, c-fms
4) Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin,

Erfindungsgemäße Antisense-Oligonucleotide der Formel I, die gegen solche Targets wirksam sind, haben beispielsweise folgende Basen-Sequenz:
a) gegen c-Ha-ras , z. B. 
5'- C A G C T G C A A C C C A G C -3'
(VIII)
c) c-myc, z.B. 
5′- G G C T G C T G G A G C G G G G C A C A C-3′
(IX)
 
5'-A A C G T T G A G G G G C A T-3'
(X)
d) c-myb, z.B. 
5′-G T G C C G G G G T C T T C G G G C -3′
(XI)
e) c-fos, z.B. 
5'-G G A G A A C A T C A T G G T C G A A A G-3'
(XII)
 
5′-C C C G A G A A C A T C A T G G T C G A A G-3′
(XIII)
 
5′-G G G G A A A G C C C G G C A A G G G G-3′
(XIV)
f) p120, z.B. 
5′-C A C C C G C C T T G G C C T C C C A C-3′
(XV)
g) EGF-Rezeptor, z.B. 
5′-G G G A C T C C G G C G C A G C G C -3′
(XVI)
 
5′-G G C A A A C T T T C T T T T C C T C C-3′
(XVII)
h) p53 Tumorsuppressor, z.B.

5′- G G G A A G G A G G A G G A T G A G G-3′
(XVIII)

5′-G G C A G T C A T C C A G C T T C G G A G-3′r
(XIX)

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Erfindungsgemäße Antisense-Oligonucleotid-Derivate, die gegen solche Targets wirksam sind, haben beispielsweise folgende Basen-Sequenz:
a) VLA-4, z.B. 
5′-G C A G T A A G C A T C C A T A T C -3′ oder
(XX)
b) ICAM, z.B. 
5'- C C C C C A C C A C T T C C C C T C T C-3'
(XXI)
 
5′-C T C C C C C A C C A C T T C C C C T C-3′
(XXII)
 
5′-G C T G G G A G C C A T A G C G A G G-3′
(XXIII)
c) ELAM-1, z. B.

5′-A C T G C T G C C T C T T G T C T C A G G -3′
(XXIV)

5'- C A A T C A A T G A C T T C A A G A G T T C-3'
(XXV)

Die Arzneimittel der vorliegenden Erfindung eignen sich beispielsweise auch zur Verhinderung der Restenose. Beispielsweise können dabei Oligonucleotid-Sequenzen zum Einsatz kommen, die gegen Targets gerichtet sind, die für Proliferation oder Migration verantwortlich sind. Solche Targets sind beispielsweise:
1) Nucleare Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, EGF, HB-EGF und TGF-β.
3) Zelluläre Rezeptoren wie beispielsweise bFGF-Rezeptor, EGF-Rezeptor und PDGF-Rezeptor.

Erfindungsgemäße Antisense-Oligonucleotide der Formel I, die gegen solche Targets wirksam sind, haben beispielsweise folgende Basen-Sequenz:
a) c-myb 
5'-G T G T C G G G G T C T C C G G G C-3'
(XXVI)
b) c-myc 
5'-C A C G T T G A G G G G C A T-3'
(XXVII)
c) cdc2-Kinase 
5'- G T C T T C C A T A G T T A C T C A-3'
(XXVIII)
d) PCNA (proliferating cell nuclear antigen of rat)

5'- G A T C A G G C G T G C C T C A A A-3'
(XXIX)

Geeignete Verabreichungsformen für Verbindungen der Formel I, in denen W und R zusammen mit dem sie tragenden Phosphonat-Rest ein Oligonucleotid bilden sind topische Applikationen, lokale Applikationen wie beispielsweise mit Hilfe eines Katheters oder aber auch Injektionen. Zur Injektion werden die Antisense-Oligonucleotid-Derivate in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst oder suspendiert werden. Die für die systemische Verabreichung bevorzugten Dosierungen betragen ca. 0,01 mg/kg bis ca. 50 mg/kg Körpergewicht und Tag.

Die Arzneimittel können z.B. auch in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

### In-vitro anti HIV Tests der Dinucleosid-o-Hydroxymethylarylphosphonate 1-3

Mit den Dinucleosid-α-Hydroxymethylarylphosphonaten 1-3 wurden in-vitro HIV-Tests durchgeführt. Als Testsystem wurden humane T-Lymphozyten (CEM/O) verwendet. Außerdem wurden die Verbindungen in einem Thymidin-Kinase defizienten T-Lymphozyten Stamm (CEM/TK⁻) getestet. Die CEM/O-Zellen wurden sowohl mit HIV-1 ald auch mit HIV-2 infiziert. Vor den Tests wurde sichergestellt, daß die zu testenden Verbindungen frei von freiem Nucleosid waren (max. 0,5 % HPLC). Die Ergebnisse der Testassays sind in Tabelle 1 aufgelistet.

Wie aus Tabelle 1 ersichtlich, zeigen alle Verbindungen hohe Aktivität sowohl gegen die HIV-1- als auch die HIV-2-Replikation.

Im Gegensatz zu den bereits in der Literatur bescheiebenen Prodrugformen wiesen die erfindungsgemäßen Verbindungen der Formel I keine cytotoxische Wirkung auf.

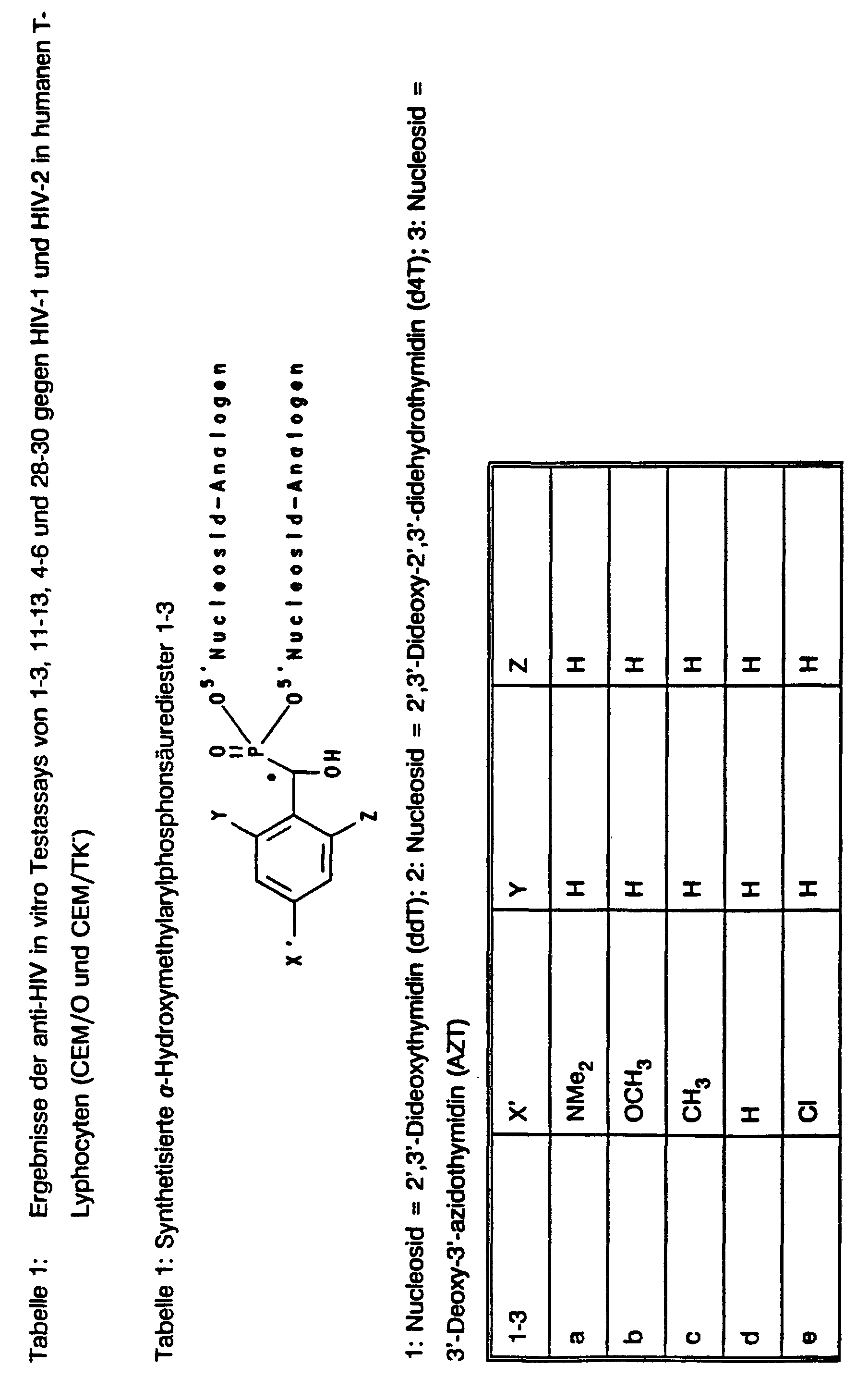

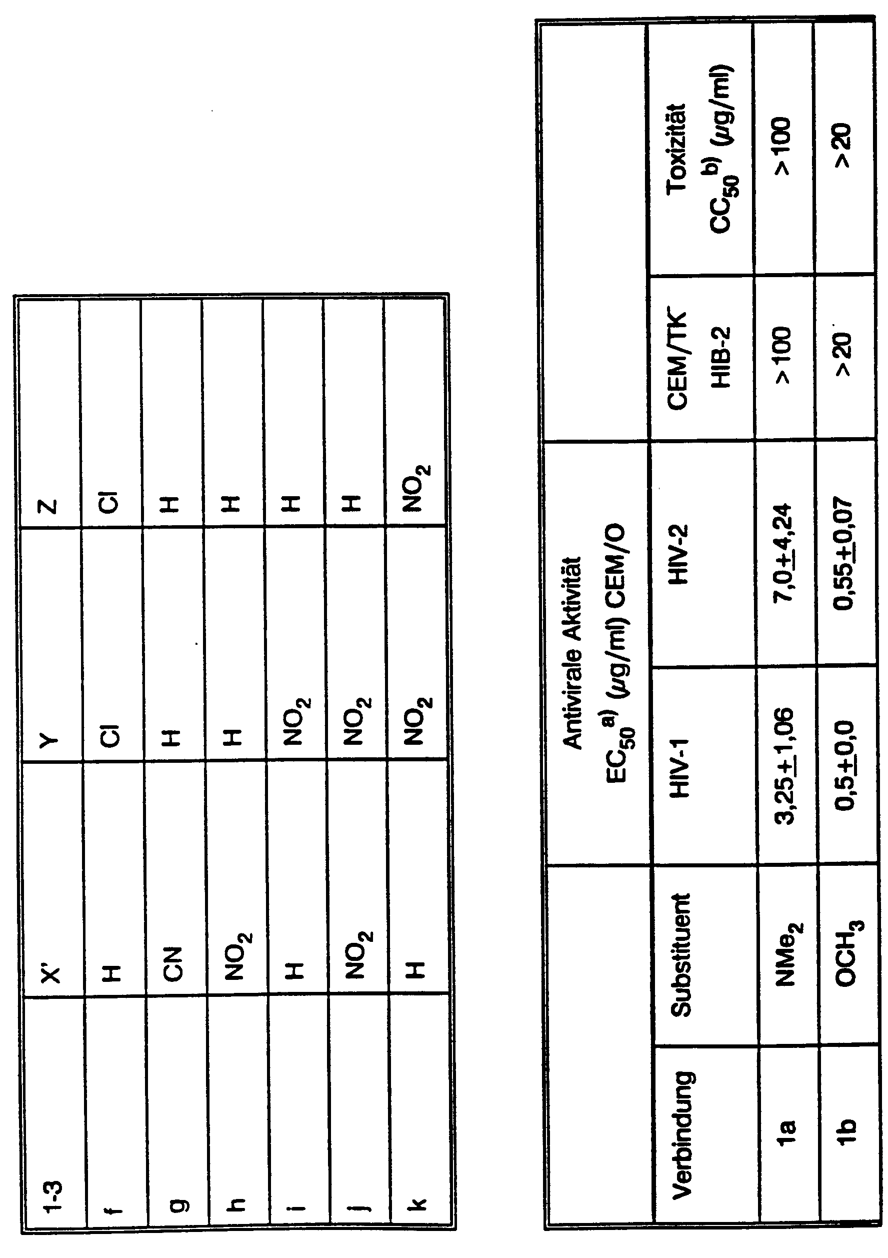

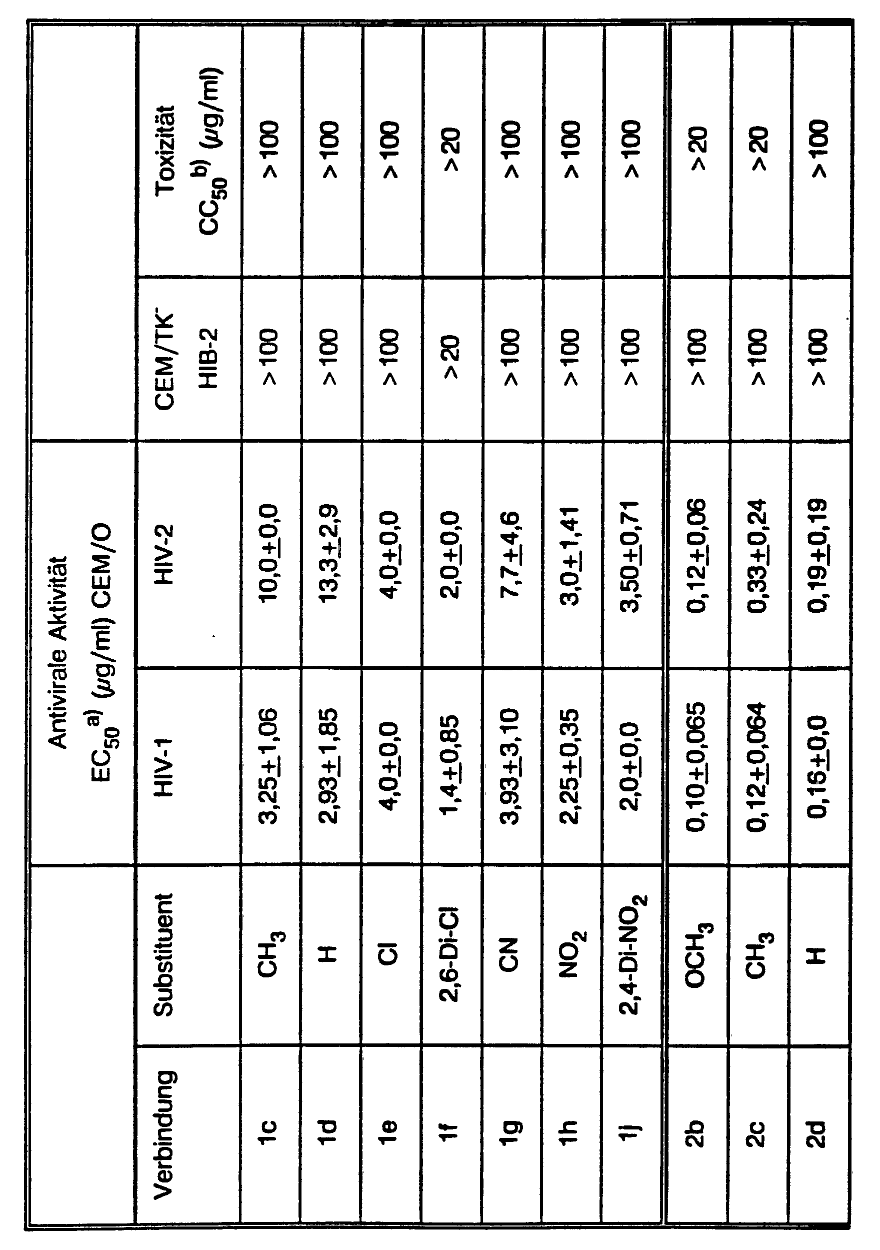

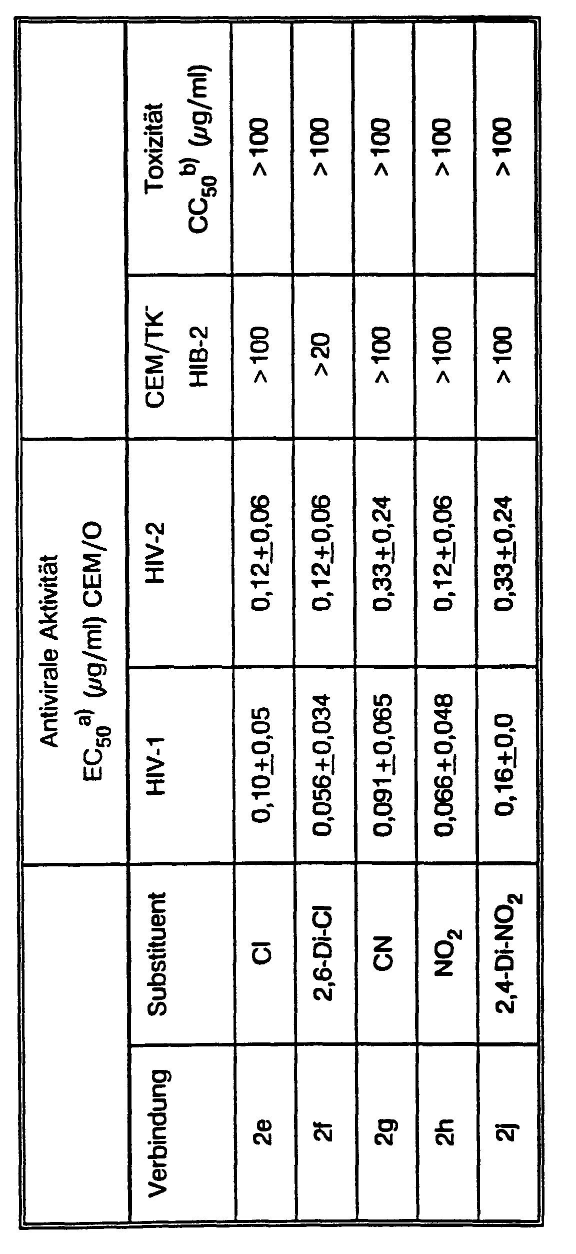

Die erfindungsgemäßen Verbindungen weisen höhere Verteilungskoeffizienten in einem Octanol/Wasser-Gemisch auf, als die ihnen zu Grunde liegenden Nucleosid-Analoga. Sie sind daher besser passiv über Biomembranen transportierbar.

Durch die nachfolgenden Ausführungsbeispiele und durch den Inhalt der Patentansprüche soll die Erfindung näher erläutert werden.

### Beispiele

### 1. Darstellung von Hydroxy-(4-Methylphenyl)-methylphosphonsäure-bis-(5'-O-2',3'-dideoxythymidin)-ester:

904 mg (4.0 mmol) 2',3'-Dideoxythymidin wurden im Hochvakuum getrocknet und anschließend in 60 ml trockenem Acetonitril gelöst. Diese Lösung wurden mit 774 mg (6.0 mmol; 1.07 ml) Diisopropylethylamin versetzt und auf 0°C im Eisbad gekühlt. Anschließend gab man portionsweise innerhalb von 15 Minuten 404 mg (2.0 mmol) Diisopropylamin-dichlorphosphin zu. Nach beendeter Zugabe ließ man unter Erwärmung auf Raumtemperatur 15 Minuten nachrühren. Bei Raumtemperatur erfolgte die Zugabe von 254 mg (4.0 mmol) Tetrazol und 80 ml Wasser. Nach 30 minütigem Rühren wurde das Lösungsmittel an der Hochvakuumanlage abkondensiert. Der Rückstand wurde am Chromatotron über Kieselgel mit Hilfe eines Gradienten aus Essigester / Methanol (0% bis 30% Methanol) aufgereinigt. Das Produkt wurde als farbloser Feststoff isoliert (797 mg [1.6 mmol]; 80% Ausbeute). 797 mg (1.6 mmol) 2',3'-ddT-H-Phosphonatdiester wurden in 40 ml trockenem Tetrahydrofuran gelöst und mit 518 mg (4.8 mmol) 4-Methylbenzaldehyd versetzt. Unter Rühren wurde zu diese Lösung 20 ml trockenes, zuvor destilliertes Triethylamin gegeben. Nach 4 Stunden bei Raumtemperatur war das Edukt abreagiert. Die abschließende Kontrolle erfolgte mit Hilfe der reversed-phase HPLC-Chromatographie. Der Reaktionsansatz wurde durch Zugabe von 20 ml Essigsäure neutralisiert und am Rotationsverdampfer bis zur Trockene eingeengt. Der Rückstand wurde am Chromatotron mit Hilfe eines Gradienten von Methylenchlorid / Methanol (0% bis 15% Methanol) aufgereinigt. Das Produkt wurde nach Lyophilisieren als farbloser Feststoff isoliert (921 mg [1.52 mmol]; 95% Ausbeute). Zur Reinigung der Verbindung für die in-vitro anti-HIV Tests wurde zusätzlich eine semipräparative HPLC-Reinigung mit einem isokratischem Elutentengemisch (30% Methanol in Acetonitril) durchgeführt.

### 2. Darstellung von Hydroxy-(4-Dimethylaminophenyl)-methylphosphonsäure-bis-(5'-O-2',3'-dideoxythymidin)-ester:

Analoge Vorschrift wie unter 1. Statt 4-Methylbenzaldehyd wurde hier 4-Dimethylaminobenzaldehyd eingesetzt. (Ausbeute 90%).

### 3. Darstellung von Hydroxy-(4-Methoxyphenyl)-methylphosphonsäure-bis-(5'-O-2',3'-dideoxythymidin)-ester:

Analoge Vorschrift wie unter 1. Statt 4-Methylbenzaldehyd wurde hier 4-Methoxybenzaldehyd eingesetzt (Ausbeute 87%).

### 4. Darstellung von Hydroxy-(phenyl)-methylphosphonsäure-bis-(5'-O-2',3'-di-deoxythymidin)-ester:

Analoge Vorschrift wie unter 1. Statt 4-Methylbenzaldehyd wurde hier Benzaldehyd eingesetzt (Ausbeute 93%).

### 5. Darstellung von Hydroxy-(4-chlorphenyl)-methylphosphonsäure-bis-(5'-O-2',3'-di-deoxythymidin)-ester:

Analoge Vorschrift wie unter 1. Statt 4-Methylbenzaldehyd wurde hier 4-Chlorbenzaldehyd eingesetzt (Ausbeute 90%).

### 6. Darstellung von Hydroxy-(4-Cyanphenyl)-methylphosphonsäure-bis-(5'-O-2',3'-di-deoxythymidin)-ester:

Analoge Vorschrift wie unter 1. Statt 4-Methylbenzaldehyd wurde hier 4-Cyanbenzaldehyd eingesetzt (Ausbeute 86%).

### 7. Darstellung von Hydroxy-(4-Nitrophenyl)-methylphosphonsaure-bis-(5'-O-2',3'-di-deoxythymidin)-ester:

Analoge Vorschrift wie unter 1. Statt 4-Methylbenzaldehyd wurde hier 4-Nitrobenzaldehyd eingesetzt (Ausbeute 85%).

### 8. Darstellung von Hydroxy-(2-Nitrophenyl)-methylphosphonsäure-bis-(5'-O-2',3'-di-deoxythymidin)-ester:

Analoge Vorschrift wie unter 1. Statt 4-Methylbenzaldehyd wurde hier 2-Nitrobenzaldehyd eingesetzt (Ausbeute 87%).

### 9. Darstellung von Hydroxy-(2.4-Dinitrophenyl)-methylphosphonsäure-bis-(5'-O-2',3'-dideoxythymidin)-ester:

Analoge Vorschrift wie unter 1. Statt 4-Methylbenzaldehyd wurde hier 2.4-Dinitroenzaldehyd eingesetzt (Ausbeute 85%).

### 10. Darstellung von Hydroxy-(9-fluorenyl)-methylphosphonsäure-bis-(5'-O-2',3'-di-deoxythymidin)-ester:

Analoge Vorschrift wie unter 1. Statt 4-Methylbenzaldehyd wurde hier 9-Fluorenon eingesetzt (Ausbeute 91%).

### 11. Darstellung von Hydroxy-(4-pyridyl)-methylphosphonsäure-bis-(5'-O-2',3'-di-deoxythymidin)-ester:

Analoge Vorschrift wie unter 1. Statt 4-Methylbenzaldehyd wurde hier 4-Pyridylaldehyd eingesetzt (Ausbeute 82%).

### 12. Darstellung von Hydroxy-(2.6-Dichlorphenyl)-methylphosphonsäure-bis-(5'-O-2',3'-dideoxythymidin)-ester:

Analoge Vorschrift wie unter 1. Statt 4-Methylbenzaldehyd wurde hier 2.4-Dichlorbenzaldehyd eingesetzt (Ausbeute 96%).

### 13. Darstellung von Hydroxy-(4-methoxyphenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 4-Methoxybenzaldehyd eingesetzt (Ausbeute 80 %).

### 14. Darstellung von Hydroxy-(4-methylphenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester eingesetzt (Ausbeute 83 %).

### 15. Darstellung von Hydroxy-(phenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde Benzaldehyd eingesetzt (Ausbeute 87 %).

### 16. Darstellung von Hydroxy-(4-chlorphenyl)-methylphosphonsäure-bis-(5'-O-2',3'-di deoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 4-Chlorbenzaldehyd eingesetzt (Ausbeute 86 %).

### 17. Darstellung von Hydroxy-(4-cyanphenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 4-Cyanbenzaldehyd eingesetzt (Ausbeute 86 %).

### 18. Darstellung von Hydroxy-(4-nitrophenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 4-Nitrobenzaldehyd eingesetzt (Ausbeute 81 %).

### 19. Darstellung von Hydroxy-(2-nitrophenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 2-Nitrobenzaldehyd eingesetzt (Ausbeute 86 %).

### 20. Darstellung von Hydroxy-(2.4-Dinitrophenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 2.4-Dinitrobenzaldehyd eingesetzt (Ausbeute 80 %).

### 21. Darstellung von Hydroxy-(4-pyridyl)-methylphosphonsäure-bis-(5'-O-2','3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 4-Pyridylaldehyd eingesetzt (Ausbeute 80 %).

### 22. Darstellung von Hydroxy-(2.6-Dichlorphenyl)-methylphosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde 2.6-Dichlorbenzaldehyd eingesetzt (Ausbeute 87 %).

### 23. Darstellung von Hydroxyheptyl-methyl-phosphonsäure-bis-(5'-O-2',3'-dideoxy-2',3'-didehydrothymidin)-ester:

Analoge Vorschrift wie unter 1. Statt 2',3'-ddT-H-Phosphonatdiester wurde hier d4T-H-Phosphonatdiester und statt 4-Methylbenzaldehyd wurde Octanal eingesetzt (Ausbeute 75 %).

### 24. Darstellung von Hydroxy-(4-nitrophenyl)-methylphosphonsäure-bis-(5'-O-2',3'-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde 4-Nitrobenzaldehyd eingesetzt (Ausbeute 96 %).

### 25. Darstellung von Hydroxy-(2-nitrophenyl)-methylphosphonsäure-bis-(5'-O-2',3'-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde 2-Nitrobenzaldehyd eingesetzt (Ausbeute 92 %).

### 26. Darstellung von Hydroxy-(2.6-di-nitrophenyl)-methylphosphonsäure-bis-(5'-O-2',3 '-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde 2.6-Dinitrobenzaldehyd eingesetzt (Ausbeute 88 %).

### 27. Darstellung von Hydroxy-(2.4-di-nitrophenyl)-methylphosphonsäure-bis-(5'-O-2',3 '-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde 2.4-Dinitrobenzaldehyd eingesetzt (Ausbeute 90 %).

### 28. Darstellung von Hydroxy-(4-pyridyl)-methylphosphonsäure-bis-(5'-O-2',3'-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde 4-Pyridylaldehyd eingesetzt (Ausbeute 96 %).

### 29. Darstellung von Hydroxyheptyl-methylphosphonsäure-bis-(5'-O-2',3'-dideoxy-3'-azidothymidin)-ester:

Analoge Vorschrift wie unter 1. Statt 2',3'-ddT-H-Phosphonatdiester wurde hier AZT-H-Phosphonatdiester und statt 4-Methylbenzaldehyd wurde Octanal eingesetzt (Ausbeute 96 %).

### 30. Darstellung von Hydroxy-(2.6-dinitrophenyl)-methylphosphonsäure-(5'-O-2',3'-dideoxy-3'-azidothymidin)-(5'-O-2',3'-dideoxythymidin)-ester:

Analoge Vorschrift wie unter 1. Statt 2',3'-ddT-H-Phosphonatdiester wurde hier ddT/AZT-H-Phosphonatdiester und statt 4-Methylbenzaldehyd wurde 2.6-Nitrobenzaldehyd eingesetzt (Ausbeute 88 %).

### 31. Darstellung von Hydroxy-(4-nitrophenyl)-methylphosphonsäure-(5'-O-2',3'-dideoxy-3'-azidothymidin)-(5'-O-2',3'-dideoxy-2',3'-didehydrothymidin)-ester:

Analoge Vorschrift wie unter 1. Statt 2',3'-ddT-H-Phosphonatdiester wurde hier d4T/AZT-H-Phosphonatdiester und statt 4-Methylbenzaldehyd wurde 4-Nitrobenzaldehyd eingesetzt (Ausbeute 96 %).

### 32. Darstellung von Hydroxy-(2.6-dinitrophenyl)-methylphosphonsäure-(5'-O-2',3'-dideoxy-3'-azidothymidin)-(5'-O-2,3'-dideoxy-2',3'-didehydrothymidin)-ester:

Analoge Vorschrift wie unter 1. Statt 2',3'-ddT-H-Phosphonatdiester wurde hier d4T/AZT-H-Phosphonatdiester und statt 4-Methylbenzaldehyd wurde 2.6-Dinitrobenzaldehyd eingesetzt (Ausbeute 87 %).

### 40. Darstellung von Hydroxy-(2-nitrophenyl)-methylphosphonsäure-(5'-O-2',3'-dideoxy-thymidin)-(5'-O-2',3'-dideoxy-2',3'-didehydrothymidin)-ester:

Analoge Vorschrift wie unter 1. Statt 2',3'-ddT-H-Phosphonatdiester wurde hier d4T/ddT-H-Phosphonatdiester und statt 4-Methylbenzaldehyd wurde 2-Nitrobenzaldehyd eingesetzt (Ausbeute 91 %).

### 41. Darstellung von Hydroxy-(2-nitrophenyl)-methylphosphonsäure-(5'-O-thymidin)-(5'-O-2',3'-dideoxy-2',3'-didehydrothymidin)-ester:

Analoge Vorschrift wie unter 1. Statt 2',3'-ddT-H-Phosphonatdiester wurde hier d4T/T-H-Phosphonatdiester und statt 4-Methylbenzaldehyd wurde 2-Nitrobenzaldehyd eingesetzt (Ausbeute 85 %).

### 42. Darstellung von Hydroxy-(4-chlorphenyl)-methylphosphonsäure-bis-(5'-O-3'-O-levulinylthymidin)-ester:

Analoge Vorschrift wie unter 1. Statt 2',3'-ddT-H-Phosphonatdiester wurde hier 3'-Levulinylthymidin-H-Phosphonatdiester und statt 4-Methylbenzaldehyd wurde 4-Chlorbenzaldehyd eingesetzt (Ausbeute 93 %).

### 43. Darstellung von Hydroxy-(4-chlorphenyl)-methylphosphonsäure-bis-(5'-O-3'-O-t-butyldimethylsilylthymidin)-ester:

Analoge Vorschrift wie unter 1. Statt 2',3'-ddT-H-Phosphonatdiester wurde hier 3'-Levulinylthymidin-H-Phosphonatdiester und statt 4-Methylbenzaldehyd wurde 4-Chlorbenzaldehyd eingesetzt (Ausbeute 85 %).

### 44. Darstellung von Hydroxy-(4-chlorphenyl)-methylphosphonsäure-bis-(5'-O-3'-O-acetylthymidin)-ester:

Analoge Vorschrift wie unter 1. Statt 2',3'-ddT-H-Phosphonatdiester wurde hier 3'-Acetylthymidin-H-Phosphonatdiester und statt 4-Methylbenzaldehyd wurde 4-Chlorbenzaldehyd eingesetzt (Ausbeute 75 %).

### 45. Darstellung von Hydroxy-(4-methoxyphenyl)-methylphosphonsäure-bis-(5'-O-3'-O-acetylthymidin)-ester:

Analoge Vorschrift wie unter 1. Statt 2',3'-ddT-H-Phosphonatdiester wurde hier 3'-Acetylthymidin-H-Phosphonatdiester und statt 4-Methylbenzaldehyd wurde 4-Methoxybenzaldehyd eingesetzt (Ausbeute 70 %).

### 46. Darstellung von Hydroxy-(4-chlorphenyl)-methylphosphonsäure-bis-(5'-O-thymidin)-ester: Die Darstellung erfolgte aus dem unter 5a) beschriebenen 3'-O-Levulinyl-geschützten Derivat nach Standard-Bedingungen mit Hilfe von 5 Äquivalenten Hydrazin Hydrat in Pyridin/Essissäure 4:1 innerhalb von 15 Minuten bei Raumtemperatur.

### 47. Darstellung von Di-(5'-O-2',3'-dideoxy-2',3'-didehydrothymidin)-phosphit:

Analoge Vorschrift wie unter 1. Statt 2',3'-Dideoxythymidin wurde 2',3'-Dideoxy-2',3'-didehydrothymidin eingesetzt (Ausbeute 75 %).

### 48. Darstellung von Di-(5'-O-2',3'-dideoxy-3'-azidothymidin)-phosphit:

Analoge Vorschrift wie unter 1. Statt 2',3'-Dideoxythymidin wurde 2',3'-Dideoxy-3'-azidothymidin eingesetzt (Ausbeute 85 %).

### 49. Darstellung von Di-(5'-O-3'-levulinylthymidin)-phosphit:

Analoge Vorschrift wie unter 1. Statt 2',3'-Dideoxythymidin wurde 3'-Levulinylthymidin eingesetzt (Ausbeute 65 %).

### 50. Darstellung von Di-(5'-O-3'-t-butyldimethylsilylthymidin)-phosphit:

Analoge Vorschrift wie unter 1. Statt 2',3'-Dideoxythymidin wurde 3'-t-Butyldimethylsilylthymidin eingesetzt (Ausbeute 65 %).

### 51. Darstellung von Di-(5'-O-3'-acetylthymidin)-phosphit:

Analoge Vorschrift wie unter 1. Statt 2',3'-Dideoxythymidin wurde 3'-Acetylthymidin eingesetzt (Ausbeute 86 %).

Die Reaktionen mit den stark Akzeptor-substituierten Benzaldehyden (4-Nitro-, 2-Nitro-, 2.6-Dinitro- und 2.4-Dinitrobenzaldehyd) ließen sich auch mit dem chiralen Chinin als Base durchführen. Die Reaktionen mit den Donor-substituierten Benzaldehyden (4-Dimethylamino-, 4-Methoxy-, 4-Methyl- und Benzaldehyd) wurden alternativ zu den oben beschriebenen Versuchen auch in reinem Triethylamin unter Erhitzen durchgeführt.

### 52. Darstellung von 5'-O-(4,4'-Dimethoxytrityl)-thymidylyl-(3'5')-thymidin-3'-((-O-tr iethylsiloxy)-2-nitrobenzyl)-phosphonat (TES-geschütztes Hydroxy-3'-OH-phosphonat-Dimer D):

### a) Darstellung von 5'-O-(4,4'-Dimethoxytrityl)-thymidylyl-(3'5')-3'-O-levulinylthymidin-3'-H-phosphonat:

(H-Phosphonat-Dimer A)
1,9 g (2,7 mmol; 1,1 eq.) 5'-O-(4,4'-Dimethoxytrityl)-thymidylyl-3'-H-phosphonat wurden im Hochvakuum getrocknet und in 30 ml trockenem Pyridin gelöst. Zu dieser Lösung wurden 828 mg (2,4 mmol; 1,0 eq.) vorgetrocknetes 3'-O-Levuinylthymidin gegeben. Anschließend wurden 899 ml frisch destilliertes Pivaloylchlorid zugetropft und bei Raumtemperatur weitergerührt. Nach 8 min. wurde mit 150 ml Methylenchlorid verdünnt und im Scheidetrichter mit 150 ml 5%-iger
Natriumhydrogencarbonat-Lösung extrahiert. Nach weiterem zweimaligem Extrahieren mit je 150 ml Methylenchlorid wurde über Natriumsulfat getrocknet, vom Trockenmittel abfiltriert und am Rotationsverdampfer bis zur Trockene eingeengt. Das Rohprodukt wurde mittels Flashchromatographie gereinigt. Der Gradient des Laufmittels Essigester / Methanol (+ Zusatz von 0,1 % Essigsäure) wurde von 0 % auf 5 % Methanol erhöht.
Das Produkt wurde als gelber Feststoff isoliert (1,972 g ; 2,12 mmol ; 78 %).

### b) Darstellung von 5'-O-(4,4'-Dimethoxytrityl)-thymidylyl-(3'5')-3'-O-levulinylthymidin-3'-((-hydroxy)-2-nitrobenzyl)-phosphonat:

(α-Hydroxyphosphonat-Dimer B)
1,5 g (1,6 mmol; 1 eq.) des H-Phosphonat-Dimers A wurden vorgetrocknet in 20 ml trockenem Methylenchlorid gelöst. Diese Lösung wurde mit 725 mg (4,8 mmol; 3 eq.) vorgetrocknetem 2-Nitrobenzaldehyd und anschließend mit 40 ml Triethylamin versetzt. Nach achtstündigem Rühren bei Raumtemperatur wurde mit Essigsäure neutralisiert und die Lösung direkt mittels Flashchromatographie gereinigt. Der Gradient des Laufmittels Methylenchlorid / Methanol (+ Zusatz von 0,1 % Essigsäure) wurde von 0 % auf 5 % Methanol erhöht.
Das Produkt ist ein farbloser Feststoff (1,374 g ; 1,27 mmol ; 79 %).

### c) Darstellung von 5'-O-(4,4'-Dimethoxytrityl)-thymidylyl-(3'5')-3'-O-levulinylthymidin-3'-((-O-triethylsiloxy)-2- nitrobenzyl)-phosphonat:

(TES-geschütztes Hydroxyphosphonat-Dimer C)
1,1 g (1,01 mmol ; 1 eq.) -Hydroxyphosphonat-Dimer B wurden vorgetrocknet in 20 ml trockenem Pyridin gelöst. Zu dieser Lösung wurden 914 mg (6,07 mmol ; 1,02 ml; 6 eq.) Triethylsilylchlorid getropft und bei Raumtemperatur gerührt. Nach siebenstündigem Rühren wurde am Rotationsverdampfer bis zur Trockene eingeengt.
Das Rohprodukt wurde mittels Flashchromatographie gereinigt. Der Gradient des Laufmittels Essigester / Methanol wurde von 0 % auf 4 % Methanol erhöht.
Das Produkt ist ein hellgelber Feststoff (1,13 g ; 0,95 mmol ; 93 %).

### d) Darstellung von 5'-O-(4,4'-Dimethoxytrityl)-thymidylyl-(3'5')-thymidin-3'-((- O-triethylsiloxy)-2-nitrobenzyl)-phosphonat (D):

1,1 g (0,92 mmol) TES-geschütztes Hydroxyphosphonat-Dimer C wurden in 10 ml Pyridin gelöst und mit 10 ml einer Lösung aus 3 ml Hydrazinhydrat (24 % in Wasser), 6,92 ml Pyridin und 4,61 ml Essigsäure versetzt. Nach dreiminütigem Rühren bei Raumtemperatur wurde die Lösung auf 0° C gekühlt und mit 100 ml Wasser und 100 ml Essigester verdünnt. Nach dem Trennen der Phasen im Scheidetrichter wurde die organische Phase einmal mit 25 ml einer 5%-igen Natriumhydrogencarbonat-Lösung gewaschen und anschließend über Natriumsulfat getrocknet. Nach dem Abtrennen vom Trockenmittel wurde am Rotationsverdampfer bis zur Trockene eingeengt. Das Rohprodukt wurde mittels Flashchromatographie gereinigt. Der Gradient des Laufmittels Methylenchlorid / Methanol wurde von 0 % auf 7 % Methanol erhöht.
Das Produkt wurde als hellgelber Feststoff isoliert (858 mg ; 0,78 mmol ; 85 %).

### 53. Darstellung von 5'-O-(4,4'-Dimethoxytrityl)-thymidylyl-(3'5')-thymidin-3'-O-(β-cy anoethyl-diisopropylaminophosphoramidit)-((-O-triethylsiloxy)-2-n itrobenzyl)-phosphonat

(TES-geschütztes Hydroxy-3'-phosphoramidit-phosphonat-Dimer E)
230 mg (0,21 mmol ; 1 eq.) TES-geschütztes Hydroxy-3'-OH-phosphonat-Dimer D wurden vorgetrocknet in 15 ml trockenem Methylenchlorid gelöst, unter Rühren mit 177 ml (1,05 mmol ; 135 mg ; 5 eq) Diisopropylethylamin und anschließend mit 70 ml (0,31 mmol ; 74,2 mg ; 1,5 eq) β-Cyanoethyl-diisopropylchlorophosphin versetzt. Es wurde 5 Stunden bei Raumtemperatur gerührt, 20 ml Essigester zugefügt und am Rotationsverdampfer bis zur Trockene eingeengt. Anschließend wurde zweimal mit je 20 ml 2%iger Natriumhydrogencarbonat-Lösung gefolgt von gesättigter
Kochsalz-Lösung extrahiert. Die organische Phase trocknete man über Natriumsulfat. Nach Filtration wurde der Rückstand am Rotationsverdampfer bis zur Trockene eingeengt.
Das Rohprodukt wurde mittels Flashchromatographie gereinigt. Als Laufmittel wurde Methylenchlorid / Acetonitril 1:1 (+ Zusatz von 1% Triethylamin) verwendet. Das Produkt ist ein hellgelber Feststoff (123 mg ; 0,095 mmol ; 45%).

### 54. Darstellung von 5'-O-(4,4'-Dimethoxytrityl)-thymidylyl-(3'→5')-thymidin-3'-O-succinyl-((-O-triethylsiloxy)-2-nitrobenzyl)-pho sphonat

(TES-geschütztes Hydroxy-3'-succinylphosphonat-Dimer F)
200 mg (0,18 mmol; 1,4 eq) TES-geschütztes Hydroxy-3'-OH-Phosphonat-Dimer D wurden vorgetrocknet in 2 ml trockenem Pyridin gelöst. Anschließend wurden nacheinander 24,4 mg (0,2 mmol) 4-Dimethylaminopyridin und 20,0 mg (0,2 mmol) Bernsteinsäureanhydrid zugegeben und unter Rühren bei Raumtemperatur 4 Stunden reagieren lassen. Es wurden 45 ml Wasser zugegeben und nach 10 minütigem Nachrühren zur Trockene am Rotationsverdampfer eingeengt. Der Rückstand wurde in 15 ml Methylenchlorid aufgenommen und einmal mit 8 ml kalter 10%iger Zitronensäure und zweimal mit je 8 ml kaltem Wasser extrahiert. Anschießend wurde die organische Phase über Natriumsulfat getrocknet. Nach Filtration wurde am Rotationsverdampfer bis zur Trockene eingeengt. Das Rohprodukt wurde in 3 ml Methylenchlorid aufgenommen und in 25 ml eiskaltem n-Hexan eingetropft. Das Produkt fiel als farbloser Niederschlag aus. Zur Vervollständigung der Fällung wurde die Mutterlauge bei -20°C einige Stunden gelagert, anschließend abfiltriert und getrocknet. Das Produkt ist ein farbloser Feststoff (167 mg ; 0,14 mmol ; 78 %).

### 55. Darstellung von 5'-O-(4,4'-Dimethoxytrityl)-thymidylyl-(3'→5')-thymidin-3'-O-succinyl-CPG-((α-O-triethylsiloxy)-2-nitrobenzyl)-phosphonat

(CPG-gebundenes TES-geschütztes Hydroxy-3'-succinylphosphonat-Dimer G)
In einem Pierce-Fläschchen wurden 50 mg (0,042 mmol; 1,5 eq.) TES-geschütztes Hydroxy-3'-succinylphosphonat-Dimer F in 1,5 ml trockenem Dimethylformamid gelöst und mit 13,41 mg (0,042 mmol; 1,5 eq.) O-Benzotriazol-1-yl-N,N,N',N',-tetramethyluronium-tetrafluorobora t (TBTU) und 4,2 ml (0,033 mmol ; 3,84 mg ; 1,2 eq.) N-Ethylmorpholin versetzt. Anschließend wurden 370 mg CPG-Träger zugegeben und weitere 4 Stunden bei Raumtemperatur geschüttelt. Es wurde in eine Fritte überführt, mit Methanol und Methylenchlorid gewaschen, wieder in das Pierce-Fläschchen überführt und mit 1,5 ml Capping-Reagenz versetzt. Es wurde eine weitere Stunde geschüttelt, erneut in eine Fritte überführt, abfiltriert, mit Methanol, Methylenchlorid, Tetrahydrofuran und Diethylether gewaschen und bei 40° C im Ölpumpenvakuum getrocknet.
Beladung des Trägers : 47,12 mmol/g

### 56. Herstellung eines Oligonucleotids der Formel

TTTTTTTTT(pp)T

(pp bedeutet eine α-Hydroxy(o-nitrophenyl)methylphosphonat-Brücke)
Der CPG-Träger aus Beispiel 55, der 1 *µ*mol des Dinucleotids über das 3'-Ende gebunden enthält, wird nacheinander mit folgenden Reagenzien behandelt:
1. Acetonitril abs.
2.2 % Dichloressigsäure in Dichlormethan
3. Acetonitril abs.
4. 10 *µ*mol 5'-O-Dimethoxytritylthymidin-3'-phosphorigsäure-β-cyanoethylester-diisopropylamidit und 40 *µ*mol Tetrazol in Acetonitril abs.
5. Acetonitril
6. 20 % Acetanhydrid in THF mit 40% Lutidin und 10 % Dimethylaminopyridin
7. Acetonitril
8. Jod (1.3 gr in THF/Wasser/Pyridin; 70:20:5=v:v:v)

Die Schritte 1 bis 8 , nachfolgend ein Reaktionszyklus genannt, werden zum Aufbau des Decathymidylat-Derivats 7-mal wiederholt. Nach abgeschlossener Synthese erfolgt die Abspaltung der Dimethoxytritylgruppe wie in den Schritten 1 bis 3 beschrieben. Durch Behandlung mit Ammoniak wird das Oligonucleotid vom Träger gespalten und zugleich werden die β-Cyanoethyl-Gruppen eliminiert. Die Abspaltung der Silylschutzgruppe erfolgt durch Behandlung mit 80 %-iger Esisigsäure. Das erhaltene Rohprodukt an DecathymidylatDerivat, das am 3'-Ende eine (-Hydroxy-o-nitro-phenyl-methylphosphonat)-Internucleotid-Bin dung enthält, wird durch Polyacrylamid-Gelelektrophorese oder HPLC gereinigt.

### 57. Herstellung eines Oligonucleotids der Formel

T(pp)TTTTTTTTT

Kommerziell erhältlicher CPG-Trägers , der 1 *µ* Mol des 5'-O-Dimerthoxytritylthymidins über das 3'-Ende gebunden enthält, wird nacheinander mit folgenden Reagenzien behandelt:
1. Acetonitril abs.
2.2% Dichloressigsäure in Dichlormethan
3. Acetonitril abs.
4. 10 *µ*mol 5'-O-Dimethoxytritylthymidin-3'-phosphorigsäure-β-cyanoethylester-diisopropylamidit und 40 *µ*mol Tetrazol in Acetonitril abs.
5. Acetonitril
6. 20 % Acetanhydrid in THF mit 40% Lutidin und 10 % Dimethylaminopyridin
7. Acetonitril
8. Jod (1.3 gr in THF/Wasser/Pyridin; 70:20:5=v:v:v)

Die Schritte 1 bis 8 , nachfolgend ein Reaktionszyklus genannt, werden zum Aufbau des Decathymidylat-Derivats 7-mal wiederholt. Im letzten Zyklus wird anstelle des Monomers in Schritt 4 das entsprechende Dinucleotid, das wie in Beispiel 53 hergestellt wurde, eingesetzt. Nach abgeschlossener Synthese erfolgt die Abspaltung der Dimethoxytritylgruppe wie in den Schritten 1 bis 3 beschrieben. Durch Behandlung mit Ammoniak wird das Oligonucleotid vom Träger gespalten und zugleich werden die β-Cyanoethyl-Gruppen eliminiert. Die Abspaltung der Silylschutzgruppe erfolgt durch Behandlung mit 80 %-iger Esisigsäure. Das erhaltene Rohprodukt an DecathymidylatDerivat, das am 5'-Ende eine (-Hydroxy-o-nitrophenyl-methylphosphonat)-Internucleotid-Bind ung enthält, wird durch Polyacrylamid-Gelelektrophorese oder HPLC gereinigt.

### 58. Herstellung eines Oligonucleotids der Formel

T(pp)TTTTTTTT(pp)T

(pp bedeutet jeweils eine α-Hydroxy(o-nitrophenyl)methylphosphonat-Brücke)
Die Synthese erfolgt analog wie in Beispiel 56 beschrieben ausgehend vom T(pp)T-CPG-Trägers aus Beispiel 55, der 1 *µ*mol des Dinucleotids über das 3'-Ende gebunden enthält. Im letzten Zyklus wird anstelle des Monomers in Schritt 4 das entsprechende Dinucleotid, das wie in Beispiel 53 hergestellt wurde, eingesetzt. Nach abgeschlossener Synthese erfolgt die Abspaltung der Dimethoxytritylgruppe wie in den Schritten 1 bis 3 beschrieben. Durch Behandlung mit Ammoniak wird das Oligonucleotid vom Träger gespalten und zugleich werden die β-Cyanoethyl-Gruppen eliminiert. Die Abspaltung der Silylschutzgruppe erfolgt durch Behandlung mit 80 %-iger Esisigsäure. Das erhaltene Rohprodukt an DecathymidylatDerivat, das am 3'- und am 5'-Ende jeweils eine (-Hydroxy-o-nitrophenyl-methylphosphonat)-Internucleotid-Bind ung enthält, wird durch Polyacrylamid-Gelelektrophorese oder HPLC gereinigt.

### 59. Herstellung von GCAGGAGGATGCTGAGGAGG(pp)C (HSV Target)

Die Herstellung erfolgt analog wie in Beispiel 56 beschrieben, wobei man anstelle von T(pp)T-CPG von entsprechendem G(pp)C-CPG-Träger ausgeht. In den Kondensationsreaktionen werden im Schritt 4 jeweils die der Sequenz entsprechenden Monomerbaustein des Desoxyadenosins, Desoxyguanosins oder des Desoxycytidins eingesetzt. Man bevorzugt solche kommerziell erhältliche Bausteine, die schnell abspaltbare Schutzgruppen besitzen (^{R}Expedite Fast Deprotecting Amidites; Fa. Millipore, Eschborn).

### 60. Herstellung von G(pp)CAGGAGGATGCTGAGGAGG(pp)C

Die Herstellung erfolgt analog wie in Beispiel 5 beschrieben, wobei man im letzten Kondensationsschritt anstelle des T(pp)T-Phosphoramidits das entsprechende G(pp)C-Phosphoramidit einsetzt.

### 61. Herstellung von G(pp)CAGGAGGATG(pp)CTGAGGAGG(pp)C

Die Herstellung erfolgt analog wie in Beispiel 5 beschrieben, wobei man im achten und im letzten Kondensationsschritt jeweils das entsprechende G(pp)C-Phosphoramidit einsetzt.

### 62. Herstellung von G(pp)CGGGGCTCCATGGGGGTC(pp)G

Die Herstellung erfolgt analog wie in Beispiel 60 beschrieben, wobei man anstelle von G(pp)C-CPG von entsprechendem C(pp)G-CPG-Träger ausgeht.

### 63. Herstellung von C(pp)GAGAACATCATGGTC(pp)G (c-fos Target)

Die Herstellung erfolgt analog wie in Beispiel 62 beschrieben, wobei man im letzten Zyklus das entsprechende C(pp)G-Phosphoramidit einsetzt.

### 64. Charakterisierung der Oligonucleotide

Die Charakterisierung erfolgt mit Hilfe der HPLC, Polyacrylamid-Gelelektrophorese (PAGE) und Negativionen Elektrospray Massenspektrometrie (ES-MS⁻). Die Produkte werden wie oben beschrieben gereingt und zeigen danach bei der PAGE (20% Acrylamid, 2% Bisacrylamid und 7 M Harnstoff) eine einheitliche Bande. Die HPLC erfolgt auf Reversed Phase Säulen RP-18 der Fa. Merck oder auf einer PA-100-Säule der Fa. Dionex.
Für die ES-MS⁻ werden die Oligonucleotide durch Ammoniumacetat-Fällung oder andere Umsalzung in die Ammonium-Salze übergeführt. Die Probenaufgabe efolgt aus einer Lösung in Acetonitril/Wasser (1:1) mit 5 OD₂₆₀/ml Oligomer. Die Genauigkeit der Methode liegt bei ca. ± 1.5 Dalton.

### 65. Bestimmung der Stabilität und Zellaufnahme nach radioaktiver Markierung

### Radioaktive Markierung:

Eine allgemein anwendbare Markierung mit ³⁵S besteht darin, daß man bei der Synthese des Oligonucleotids mindestens eine Oxidation im DNA-Synthesezyklus (Schritt 20 in Beispiel 11) mit elemntarem ³⁵Schwefel durchführt. Oligonucleotide , die eine freie 5'-Hydroxygruppe haben, können mit Hilfe der Polynucleotid-Knase nach an sich bekanneten Methoden mit ³²P oder ³⁵S markiert werden. Oligonucleotide , die eine freie 3'-Hydroxygruppe tragen, können nach bekannter Art mit 3'-terminaler Transferase markiert werden. Als Beispiel ist hier die 5'-Markierung des DNA-Teils wiedergegen: Das Oligonucleotid mit einer freien 5'-Hydroxygruppe (500 pmol) wird in 425 *µ*l Wasser gelöst, diese Lösung auf 90°C erhitzt und abgeschreckt. Dann gibt man 50 *µ*l 10 x Kinase-Puffer und 50 *µ*l ³²P Gamma-ATP (6000 Ci/ mmol) bzw. ^{35S-} Gamma-ATP zu und inkubiert 1 Stunde bei 37°C. Die Reaktion wird durch Zugabe von 0.5 M EDTA-Lösung gestoppt. Die Entsalzung erfolgt mit Hilfe einer NAP^{R}-Säule der Firma Pharmacia.

Untersuchung der Stabiltät des Oligomers im Medium mit Zellen:

Der Überstand 1 (10 *µ*l) wird mit 5 *µ* l 80 % Formamid (mit XC und BB) gemischt, auf 95°C erhitzt (5 Minuten) und auf ein Polyacrylamidgel (20 % Acrylamid, 7 M Harnstoff) geladen. Nach der Entwicklung des Gels im elektrischen Feld ordnet man die Banden auf dem Gel mittels Autoradiographie dem "Stabilen Oligomer" bzw. die Fehlbanden dem "abgebauten Oligomer" zu. Ergebnis nach 24 Stunden Inkubationszeit: Im Vergleich zu den unmodifizierten Oligonucleotiden besitzen die Verbindugen der Formel I (W gleich Formel II, R gleich Formel II') alle eine stark verlängerte Lebensdauer.

### Bestimmung der Zellaufnahme:

Man inkubiert Vero-Zellen in 96-Napf Mikrotiterplatten in DMEM, 5 % FCS für 24 Stunden bei 37°C. Nachdem das Medium entfernt wurde, wäscht man die Zellen noch zweimal mit serumfreien DMEM. Das radioaktiv markierte Oligomer (10⁶cpm) wird mit nichtmarkiertem Oligomer auf eine Konzentration von 10 *µ*M in Serum verdünnt und die Zellen bei 37°C damit inkubiert. Nach 1, 7 und 24 Stunden werden jeweils 150 *µ*l entnommen (Bezeichnung: "Überstand 1"). Die Zellen in den Näpfen der Mikrotiterplatten werden 7-mal mit 300 *µ*l frischem Medium gewaschen und die vereinigten Waschmedien (Bezeichnung: "Überstand 2") im Szintillationszähler gemessen. Danach gibt man 100 *µ*l Trypsinlösung zu, wartet 30 Sekunden und zieht den Überstand ab. Zur Ablösung der Zellen von der Platte inkubiert man 3 Min. bei 37°C. Die abgelösten Zellen werden in 1.5 ml Eppendorf-Gefäße übergeführt und bei 2000 rpm für 6 Minuten zentrifugiert ("Überstand 3"). Die Überstände 1 (5*µ*l), 2 und 3 (0.5 ml) werden jeweils separat am Szintillationszähler gemessen. Daraus erechnet sich die Aufnahme des Oligomers in pmol per 100 000 Zellen, wobei Überstand 3 die zellgebundene Oligomerfraktion und die Summe der Überstände 1 und 2 die nicht zellgebundene Oligomerfraktion darstellen.

### 66. Bestimmung der Zellaufnahme nach Fluoreszenz-Markierung:

Man läßt die COS-Zellen bis zur Konfluenz in Dulbecco's MEM, das mit 10 % FCS supplementiert wurde, in 5 cm Petrischalen heranwachsen. Die Zellen werden zweimal mit serumfreien DMEM gewaschen. Mit Hilfe einer sterilen Nadel wird eine Fläche von ca. 1 cm² in der Mitte der Petrischale eingekratzt. In diese Fläche wird die zu untersuchende DNA-Oligomerlösung (0.1 mM) aufgebracht. Es wird bei 37°C unter CO₂ Atmosphäre inkubiert. Nach 2, 4 und 16 Stunden werden die Zellen durch Fluoreszenzmikroskopie untersucht. Dazu werden die Zellen viermal mit serumfreien DMEM gewaschen, mit einem Glasträger abgedeckt und unter dem Fluoreszenzmikroskop bzw. durch Phasenkontrast bewertet.

### 67. Bestimmung der Schmelztemperaturen:

Die Bestimmung der Schmelztemperaturen erfolgen mit Hilfe eines HP 8452A Diodenarray-Spektrophotometers, eines HP 89090A Peltier-Elements und der HP Temperature Control Software Rev. B5.1 (Fa. Hewlett Packard). Es wird in 0.5°C/min. Schritten in 10mM HEPES und 140 mM NaCI (pH 6.5) als Puffer gemessen. Die Oligomerkonzentration beträgt 0.5 bis 1.5 OD₂₆₀ pro ml.

### 68. Testung auf antivirale Aktivität in Zellkultur:

Die antivirale Aktivität der Prüfsubstanzen gegen verschiedene humanpathogene Herpesviren wird im Zellkulturtestsystem untersucht. Für den Versuch werden Affennierenzellen (Vero, 2×10⁵/ml) in serumhaltigem Dulbecco's MEM (5 % Fötales Kälberserum FCS) in 96-Napf-Mikrotiterplatten ausgesät und 24 h bei 37°C und 5 % CO₂ inkubiert. Das serumhaltige Medium wird dann abgesaugt und die Zellen werden zweimal mit serumfreiem Dulbecco's MEM (-FCS) überspült. Die Testsubstanzen werden in H₂O auf eine Konzentration von 600 *µ*M vorverdünnt und bei -18°C aufbewahrt. Für den Test erfolgen weitere Verdünnungsschritte in Dulbecco's Minimal Essential Medium (MEM). Je 100 *µ*l der einzelnen Prüfsubstanzverdünnungen werden zusammen mit 100 *µ*l serumfreiem Dulbecco's MEM (-FCS) zu den gespülten Zellen gegeben. Nach 3 h Inkubation bei 37°C und 5% CO₂ werden die Zellen mit Herpes simplex Virus Typ 1 (ATCC VR733, HSV-1 F-strain) oder mit Herpes simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 *µ*M bis 0,04 *µ*M in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden. Die Versuchsansätze werden 17 h bei 37°C und 5 % CO₂ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft. Es erfolgt daraufhin die Zugabe von FCS auf eine Endkonzentration von 4 % mit weiterer Inkubation für 55 h bei 37°C und 5% CO₂. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen. Die MIC-Werte verschiedener Oligonucleotide liegen im Bereich von 0.1 bis 80 *µ*mol/l.

### 69. Bestimmung der in vivo Aktivität gegen Viren

Für die Testung der Verbindungen in vivo werden 5 Wochen alte NMRI-Mäuse mit einem Gewicht von etwa 16 bis 18 Gramm eingesetzt. Die Mäuse werden unter herkömmlichen Bedingungen in Gruppen zu fünf Tieren und mit Futter und Wasser ad libitum gehalten. Die Infektion der Mäuse erfolgt intraperitonal mit ca. 10 bis 50 LD50 Einheiten eines HSV-Stammes (HSV "corneae"). Die Dosierung der Verbindung erfolgt zweimal täglich i. p. mit 1, 10 oder 50 mg/kg. Die Kontrolltiere erhalten eine 1%-ige Natriumchloridlösung. Die Überlebensrate der Tiere wird über eine Periode von zwei Wochen verfolgt. Bei Applikation der Antisense Oligonucleotide gibt es 1 bis 5 Überlebende, während nach Placebo-Gabe alle Tiere sterben.

### 70. Bestimmung der in vivo Aktivität: Inhibition der c-Fos Protein-Expression in der Ratte:

Die Bestimmung erfolgt wie beschrieben (Sandkühler et al. (1991) in : Proceedings of the Vlth World Congress on Pain, Charlton and Woolf, Editors; Elsevier, Amsterdam; Seite 313-318) durch Superfusion des Rückenmarks. Nach Laminektomy einer Barbiturat-anästhesierten Sprague-Dawley Ratte wird ein Zweikammer-Behältnis aus Silikon zur Aufnahme des Antisense Oligomers gebildet. Eine Kammer wird mit dem Antisense Oligonucleotid-Derivat gefüllt, während die andere Kammer mit dem Kontroll Oligomer gefüllt wird (Konzentration je 75 *µ* M). Jeweils nach einer Stunde wird das Superfusat ausgetauscht. Nach 6 Stunden Superfusion wird die c-fos Expression durch Hitzebehandlung (52°C) der Hinterläufe stimuliert. Die Inhibiton der c-fos Expression kann immunohistochemisch an entsprechenden Gewebeschnittproben nachgewiesen werden.

### 71. Darstellung von 5'-O-(4,4'-Dimethoxytriphenylmethyl)-2'-desoxythymidylyl-(3'→5')-3'-O-levulinyl-2'-desoxythymidin 3'-((α-O-tributylsiloxy)-2-nitrobenzyl)-Phosphonat:

TBS(tributylsiloxy)-geschütztes Hydroxyphosphonat-Dimer H)
1,77 g (1,636 mmol; 1 eq.) α-Hydroxyphosphonat-Dimer B wurden vorgetrocknet in 30 ml trockenem Pyridin gelöst. Zu dieser Lösung wurden 2,62 ml (9,816 mmol; 2,306 g ; 6 eq.) Chlortributylsilan getropft. Nach achtstündigem Rühren bei Raumtemperatur wurde die Reaktion durch Zugabe von Methanol abgebrochen und am Rotationsverdampfer bis zur Trockene eingeengt.
Das Rohprodukt wurde mittels Flashchromatographie gereinigt. Der Gradient des Laufmittels Essigester / Methanol wurde von 0 % auf 2 % Methanol erhöht.
Das Produkt ist ein hellgelber Feststoff (1,78 g ; 1,39 mmol ; 85 %).

### 72. Darstellung von 5'-O-(4,4'-Dimethoxytriphenylmethyl)-2'-desoxythymidylyl-(3'→5')-2'-desoxythymidin 3'-((α-O-tributylsiloxy)-2-nitrobenzyl)-Phosphonat :

(TBS-geschütztes Hydroxy-3'-OH-phosphonat-Dimer I)
1,2 g (0,94 mmol) TBS-geschütztes Hydroxyphosphonat-Dimer H wurden in 10 ml Pyridin gelöst und mit 10 ml einer Lösung aus 3 ml Hydrazinhydrat (24 % in Wasser), 6,92 ml Pyridin und 4,61 ml Essigsäure versetzt. Nach dreiminütigem Rühren bei Raumtemperatur wurde die Lösung auf 0°C gekühlt und mit 100 ml Wasser und 100 ml Essigester verdünnt. Nach dem Trennen der Phasen im Scheidetrichter wurde die organische Phase einmal mit 25 ml einer 5%-igen
Natriumhydrogencarbonat-Lösung gewaschen und anschließend über Natriumsulfat getrocknet. Das Trockenmittel wurde abfiltriert und das Filtrat am Rotationsverdampfer bis zur Trockene eingeengt.
Das Rohprodukt wurde mittels Flashchromatographie gereinigt. Der Gradient des Laufmittels Methylenchlorid / Methanol wurde von 0 % auf 5 % Methanol erhöht. Das Produkt wurde als hellgelber Feststoff isoliert (910 mg ; 0,77 mmol; 82 %).

### 73. Darstellung von 5'-O-(4,4'-Dimethoxytriphenylmethyl)-2'-desoxythymidylyl-(3'→5')-2'-desoxythymidin 3'-O-succinyl-((α-O-tributylsiloxy)-2-nitrobenzyl)-Phosphonat

(TBS-geschütztes Hydroxy-3'-succinylphosphonat-Dimer J)
120 mg (0,10 mmol; 1 eq) TBS-geschütztes Hydroxy-3'-OH-Phosphonat-Dimer I wurden vorgetrocknet in 1 ml trockenem Pyridin gelöst. Anschließend wurden nacheinander 14,8 mg (0,12 mmol; 1,2 eq.) 4-Dimethylaminopyridin und 12,1 mg (0,12 mmol; 1,2 eq.) Bernsteinsäureanhydrid zugegeben und bei Raumtemperatur 4 Stunden gerührt. Es wurden 40 ml Wasser zugegeben und nach 10 minütigem Nachrühren zur Trockene am Rotationsverdampfer eingeengt. Der Rückstand wurde in 10 ml Methylenchlorid aufgenommen und einmal mit 5 ml kalter 10%iger Zitronensäure und zweimal mit je 5 ml kaltem Wasser extrahiert. Anschießend wurde die organische Phase über Natriumsulfat getrocknet. Nach Filtration wurde am Rotationsverdampfer bis zur Trockene eingeengt.
Das Rohprodukt wurde in 3 ml Methylenchlorid aufgenommen und in 25 ml eiskaltes n-Hexan eingetropft. Das Produkt fiel als farbloser Niederschlag aus. Zur Vervollständigung der Fällung wurde die Mutterlauge bei -20°C einige Stunden gelagert, anschließend abfiltriert und getrocknet. Das Produkt ist ein farbloser Feststoff (115 mg ; 0,09 mmol ; 90 %).

### 74. Darstellung von 5'-O-(4,4'-Dimethoxytriphenylmethyl)-2'-desoxythymidylyl-(3'→5')-2'-desoxythymidin 3'-O-succinyl-CPG-((a-O-tributylsiloxy)-2-nitrobenzyl)-Phosphonat

(CPG-gebundenes TBS-geschütztes Hydroxy-3'-succinylphosphonat-Dimer K)
In einem Pierce-Fläschchen wurden 26,5 mg (0,021 mmol; 1,5 eq.) TBS-geschütztes Hydroxy-3'-succinylphosphonat-Dimer J in 0,7 ml trockenem Dimethylformamid gelöst und mit 6,74 mg (0,021 mmol; 1,5 eq.) O-Benzotriazol-1-yl-N,N,N',N',-tetramethyluronium-tetrafluoroborat (TBTU) und 2,1 ml (0,017 mmol; 1,96 mg ; 1,2 eq.) N-Ethylmorpholin versetzt. Anschließend wurden 183 mg CPG-Träger zugegeben und weitere 4 Stunden bei Raumtemperatur geschüttelt. Es wurde in eine Fritte überführt, mit Methanol und Methylenchlorid gewaschen, wieder in das Pierce-Fläschchen überführt und mit 0,7 ml Capping-Reagenz versetzt. Es wurde eine weitere Stunde geschüttelt, erneut in eine Fritte überführt, abfiltriert, mit Methanol, Methylenchlorid, Tetrahydrofuran und Diethylether gewaschen und im Ölpumpenvakuum getrocknet.
Beladung des Trägers : 33,15 mmol/g

### 75. Herstellung von CGTCCATGTCGGCAAACAGCT(pp)C (HSV Target)

Die Herstellung erfolgt analog wie in Beispiel 56 beschrieben, wobei man anstelle von T(pp)T-CPG von entsprechendem T(pp)T-CPG-Träger aus Beispiel 74 ausgeht. In den Kondensationsreaktionen werden im Schritt 4 jeweils die der Sequenz entsprechenden Monomerbaustein des Desoxyadenosins, Desoxyguanosins oder des Desoxycytidins eingesetzt. Man bevorzugt solche kommerziell erhältliche Bausteine, die schnell abspaltbare Schutzgruppen besitzen (^{R}Expedite Fast Deprotecting Amidites; Fa. Millipore, Eschbom).

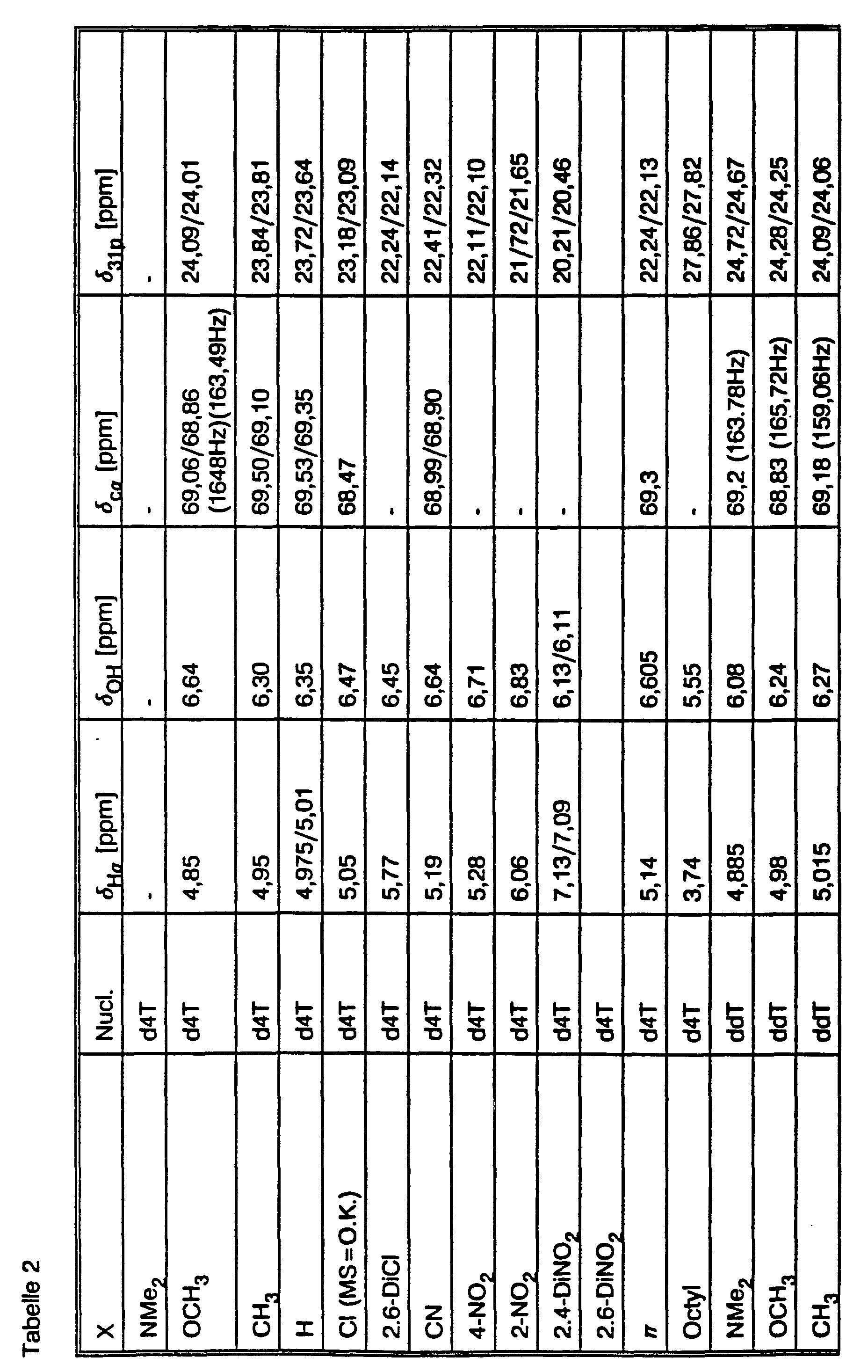

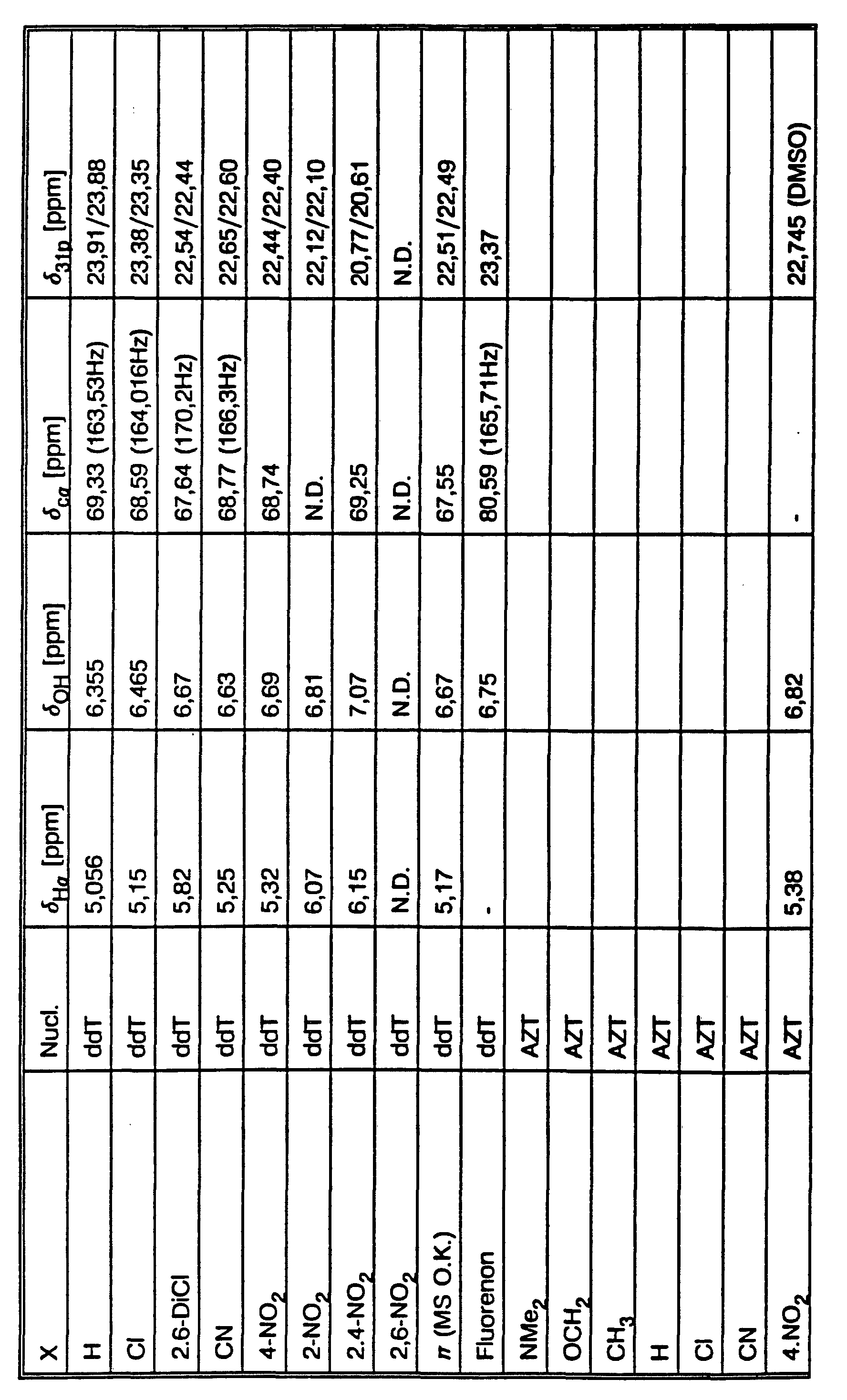

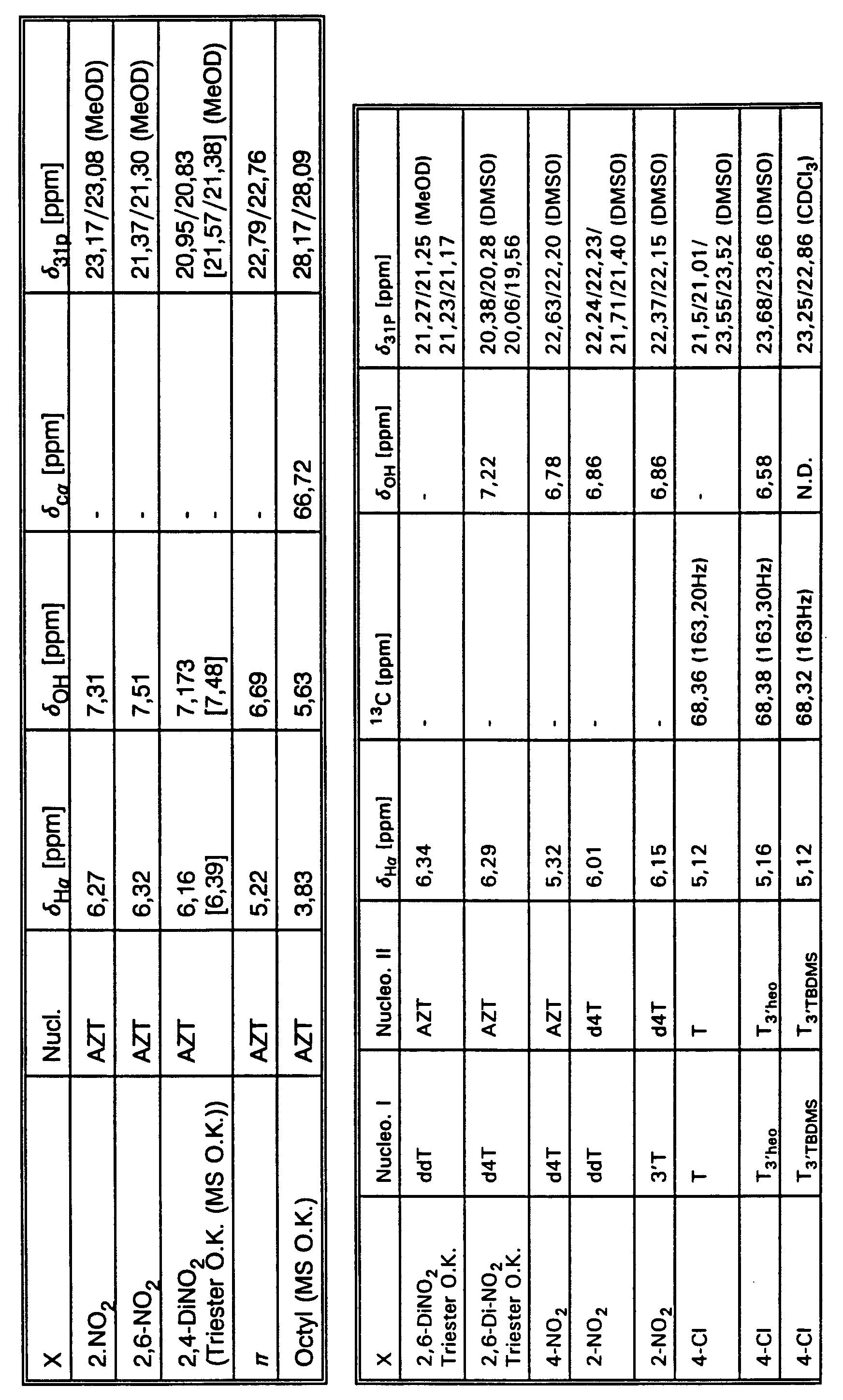

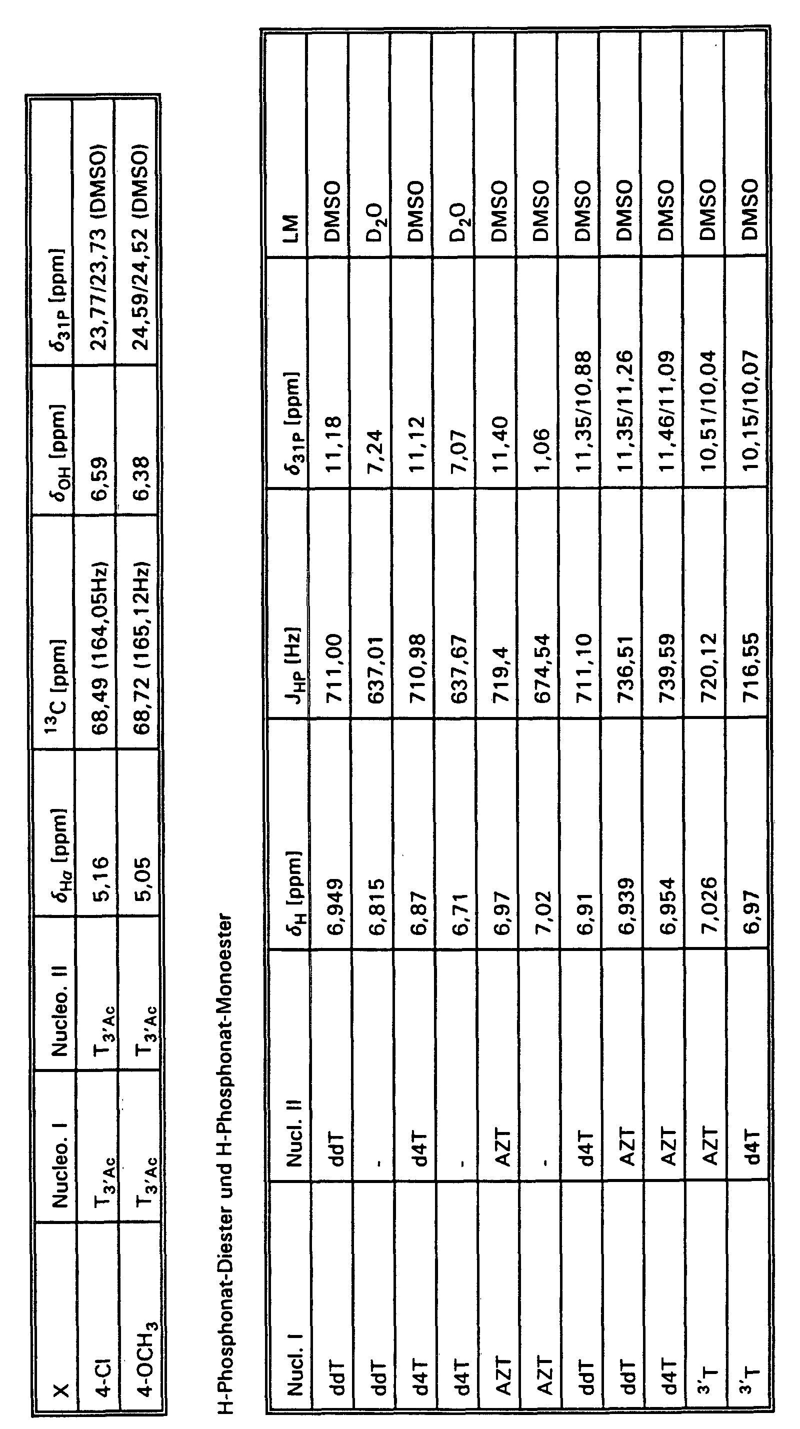

## Patentansprüche

1. Verbindungen der Formel I, 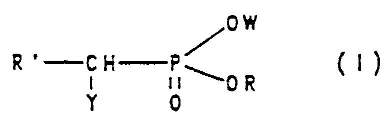 **dadurch gekennzeichnet, daß**
Y die Bedeutung von OH, SH, OAc oder SAc, mit Ac=(C1-C8)-Acyl, ggf. 1-3-fach ungesättigt, hat,
R' Aryl mit 6-14 C-Atomen, ggf. substituiert mit bis zu drei voneinander unabhängigen Resten ausgewählt aus der Gruppe (C1-C5)-Alkyl, Halogen, NO₂, CN, (C1-C6)-Alkoxy, Amino, (C1-C4)-Alkyiamino, (C1-C8)-Dialkylamino, wobei an den Arylrest auch ein (C3-C8)-Alkylenrest ankondensiert sein kann, in dem eine CH₂-Gruppe auch durch Oxy ersetzt sein kann;
Heteroaryl mit 3 bis 13 C-Atomen und bis zu drei Heteroatomen ausgewählt aus der Gruppe N, O und S;
(C1-C16)-Alkyl, verzweigt oder unverzweigt, gesättigt oder 1-3 fach ungesättigt, ggf. substituiert unabhängig voneinander mit bis zu drei Substituenten ausgewählt aus der Gruppe Halogen, CN, NO₂ und (C1-C3)-Alkoxy bedeutet,
W die Bedeutung eines pharmazeutisch wirksamen 5', 3' oder 2' Nukleosid-Analogons hat,
R die Bedeutung von W hat, wobei R und W gleich oder verschieden sein können, oder R (C1-C16)-Alkyl bedeutet, das verzweigt oder unverzweigt sein kann und ggf. substituiert ist unabhängig voneinander mit bis zu 3 Resten aus der Gruppe Halogen, CN, (C1-C8)-Acyloxy, (C1-C18)-Alkoxy oder
W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet 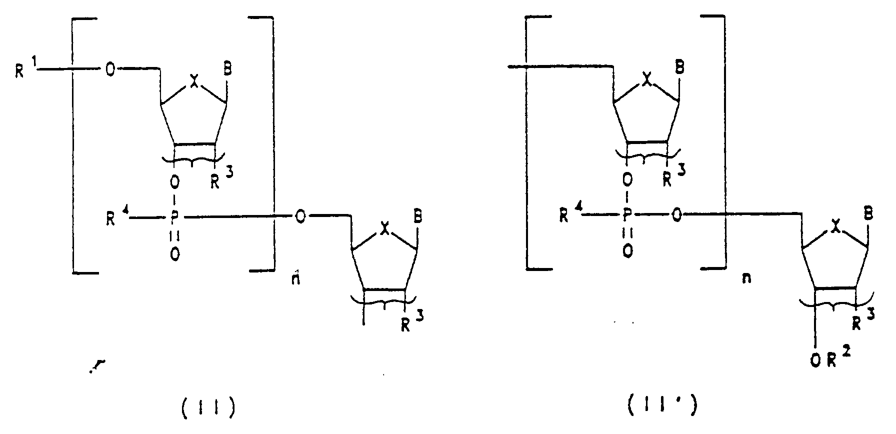
wobei X Oxy oder Sulfandiyl bedeutet,
B unabhängig voneinander eine Nukleotidbase darstellt,
n unabhängig voneinander eine ganze Zahl von 0 bis 30 bedeutet,
R¹ und R² bedeuten unabhängig voneinander H (C1-C12)-Acyl oder einen Rest der Formel 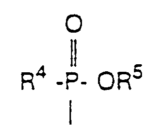
worin R⁴ O, S, CH₃ oder CHYR', mit R' und Y wie oben definiert bedeutet und R⁵ einen gegebenenfalls substituierten Alkylrest mit 1-12 C-Atomen bedeutet,
R³ bedeutet unabhängig voneinander H, O(C1-C12)-Alkyl, O(C1-C12)-Acyl, Cl, N₃, NH₂ oder NHR⁶ wobei R⁶ (C1-C3)-Alkyl oder -Acyl
und die geschweifte Klammer deutet an, daß R³ und der benachbarte Phosphonylrest sich in 2' bzw. 3' Position befinden können.

2. Verbindungen der Formel I gemäß Anspruch 1,
**dadurch gekennzeichnet, daß**
Y die Bedeutung von OH, SH, OAc oder SAc, mit Ac=(C1-C8)-Acyl, ggf. 1-3-fach ungesättigt, hat,
R' Aryl mit 6-14 C-Atomen, ggf. substituiert mit bis zu drei voneinander unabhängigen Resten ausgewählt aus der Gruppe (C1-C5)-Alkyl, Halogen, NO₂, CN, (C1-C6)-Alkoxy, Amino, (C1-C4)-Alkylamino, (C1-C8)-Dialkylamino, wobei an den Arylrest auch ein (C3-C8)-Alkylenrest ankondensiert sein kann, in dem eine CH₂-Gruppe auch durch Oxy ersetzt sein kann;
Heteroaryl mit 3 bis 13 C-Atomen und bis zu drei Heteroatomen ausgewählt aus der Gruppe N, O und S;
(C1-C16)-Alkyl, verzweigt oder unverzweigt, gesättigt oder 1-3 fach ungesättigt, ggf. substituiert unabhängig voneinander mit bis zu drei Substituenten ausgewählt aus der Gruppe Halogen, CN, NO₂ und (C1-C3)-Alkoxy bedeutet,
W die Bedeutung eines pharmazeutisch wirksamen 5', 3' oder 2' Nukleosid-Analogons hat,
R die Bedeutung von W hat, wobei R und W gleich oder verschieden sein können, oder R bedeutet oder (C1-C16)-Alkyl bedeutet, das verzweigt oder unverzweigt sein kann und ggf. substituiert ist unabhängig voneinander mit bis zu 3 Resten aus der Gruppe Halogen, CN, (C1-C8)-Acyloxy, (C1-C18)-Alkoxy oder
W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet
wobei X Oxy oder Sulfandiyl bedeutet,
B unabhängig voneinander eine Nukleotidbase darstellt,
n unabhängig voneinander eine ganze Zahl von 0 bis 30 bedeutet,
R¹ und R² bedeuten unabhängig voneinander H (C1-C12)-Acyl oder einen Rest der Formel 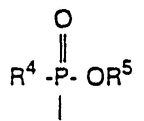
worin R⁴ O, S, CH₃ oder CHYR', mit R' und Y wie oben definiert bedeutet und R⁵ einen gegebenenfalls substituierten Alkylrest mit 1-12 C-Atomen bedeutet,
R³ bedeutet unabhängig voneinander H, O(C1-C12)-Alkyl, O(C1-C12)-Acyl, Cl, N₃, NH₂ oder NHR⁶ wobei R⁶ (C1-C3)-Alkyl oder -Acyl
und die geschweifte Klammer deutet an, daß R³ und der benachbarte Phosphonylrest sich in 2' bzw. 3' Position befinden können.

3. Verbindungen der Formel I gemäß den Ansprüche 1 oder 2,
**dadurch gekennzeichnet, daß**
Y die Bedeutung von OH, SH, OAc oder SAc hat, mit Ac=(C1-C3)-Acyl, ggf. 1-3-fach ungesättigt,
R' Aryl mit 6-14 C-Atomen, ggf. substituiert mit bis zu 3 voneinander unabhängigen Resten ausgewählt aus der Gruppe (C1-C3)-Alkyl, F, Cl, NO₂, CN, (C1-C4)-Alkoxy, Amino, (C1-C3)-Alkylamino, (C1-C6)-Dialkylamino, wobei an den Arylrest auch ein (C3-C8)-Alkylenrest ankondensiert sein kann, in dem eine CH₂-Gruppe auch durch Oxy ersetzt sein kann;
Heteroaryl mit 3 bis 6 C-Atomen und bis zu drei Heteroatomen ausgewählt aus der Gruppe N, O und S;
(C1-C8)-Alkyl, verzweigt oder unverzweigt, gesättigt oder 1-3 fach ungesättigt, ggf. substituiert unabhängig voneinander mit bis zu drei Substituenten ausgewählt aus der Gruppe Cl, CN, NO₂ und (C1-C3)-Alkoxy bedeutet.
W die Bedeutung eines pharmazeutisch wirksamen 5'-, 3' oder 2' Nukleosid-Analogons, hat,
R die Bedeutung von W hat oder (C1-C8)-Alkyl bedeutet, das verzweigt oder unverzweigt sein kann und ggf. substituiert ist mit bis zu 2 Resten aus der Gruppe Halogen, CN, (C3-C6)-Acyloxy, (C8-C18)-Alkoxy oder W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet
wobei X Oxy bedeutet,
B unabhängig voneinander eine Nukleotidbase darstellt,
n unabhängig voneinander eine ganze Zahl von 0 bis 20 bedeutet,
R¹ und R² bedeuten unabhängig voneinander H (C1-C8)-Acyl oder einen Rest der Formel 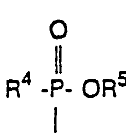
worin R⁴ O, S, CH₃ oder CHYR', mit R' und Y wie oben definiert bedeutet und R⁵ einen gegebenenfalls substituierten Alkylrest mit 1-8 C-Atomen bedeutet,
R³ bedeutet unabhängig voneinander H, O(C1-C8)-Alkyl, O(C1-C8)-Acy Cl oder N₃, und die geschweifte Klammer deutet an, daß R³ und der benachbarte Phosphonylrest sich in 2' bzw. 3' Position befinden können.

4. Verbindungen der Formel 1 gemäß einem oder mehreren der Ansprüche 1-3,
**dadurch gekennzeichnet, daß**
Y die Bedeutung von OH hat,
R' Aryl mit 6 C-Atomen, ggf. substituiert mit bis zu 3 voneinander unabhängigen Resten ausgewählt aus der Gruppe (C1-C3)-Alkyl, F, Cl, NO₂, CN, (C1-C4)-Alkoxy, Amino, (C1-C3)-Alkylamino, (C1-C6)-Dialkylamino, wobei an den Arylrest auch ein (C3-C6)-Alkylenrest ankondensiert sein kann, in dem eine CH₂-Gruppe auch durch Oxy ersetzt sein kann;
Heteroaryl mit 3 bis 6 C-Atomen und bis zu drei Heteroatomen ausgewählt aus der Gruppe N, O und S;
(C1-C8)-Alkyl, verzweigt oder unverzweigt, gesättigt oder 1-3 fach ungesättigt, vorzugsweise ungesättigt in konjugierter Form mit einer ungesättigten Bindung in alpha-Stellung, ggf. substituiert unabhängig voneinander mit bis zu drei Substituenten ausgewählt aus der Gruppe Cl, CN, NO₂ und (C1-C3)-Alkoxy bedeutet,
W die Bedeutung eines pharmazeutisch wirksamen 5'- oder 3' Nukleosid-Analogons, hat.
R die Bedeutung von W hat oder (C1-C4)-Alkyl bedeutet, das verzweigt oder unverzweigt sein kann oder
W und R bilden zusammen mit dem sie tragenden Phosphonat-Rest ein Oligonukleotid wobei W einen Rest der Formel II oder ein modifiziertes Oligonukleotid und R einen Rest der Formel II' oder ein modifiziertes Oligonukleotid bedeutet
wobei X Oxy bedeutet,
B unabhängig voneinander eine Nukleotidbase darstellt,
n unabhängig voneinander eine ganze Zahl von 0 bis 15 bedeutet,
R¹ und R² bedeuten unabhängig voneinander H (C1-C4)-Acyl oder einen Rest der Formel 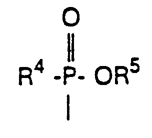
worin R⁴ O, S, CH₃ oder CHYR', mit R' und Y wie oben definiert bedeutet und R⁵ einen gegebenenfalls substituierten Alkylrest mit 1-3 C-Atomen bedeutet,
R³ bedeutet unabhängig voneinander H, O(C1-C3)-Alkyl, O(C1-C3)-Acyl, Cl oder N₃, und die geschweifte Klammer deutet an, daß R³ und der benachbarte Phosphonylrest sich in 2' bzw. 3' Position befinden können.

5. Verbindungen der Formel I gemäß einem oder mehreren der Ansprüche 1-4,
**dadurch gekennzeichnet, daß**
Y die Bedeutung von OH hat,
W die Bedeutung eines pharmazeutisch wirksamen 5' - oder 3' Nukleosid-Analogons hat,
R die Bedeutung von W hat oder (C1-C4)-Alkyl bedeutet, das verzweigt oder unverzweigt sein kann,
R' bedeutet Aryl mit 6 C-Atomen, ggf. substituiert mit bis zu 3 voneinander unabhängigen Resten ausgewählt aus der Gruppe Cl, NO₂, CN, (C1-C3)-Alkoxy, Amino und (C1-C3)-Alkylamino.

6. Verfahren zur Herstellung von Verbindungen gemäß einem oder mehreren der Ansprüche 1-5, **dadurch gekennzeichnet, daß**
a) eine Verbindung der Formel III mit einer Verbindung der Formel IV umgesetzt wird, 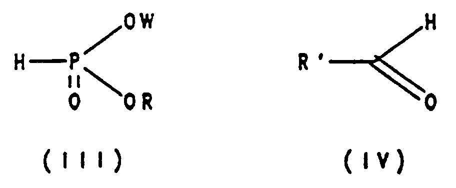 oder daß
b) eine Verbindung der Formel V in beliebiger Reihenfolge und unter Anwendung eines Kondensationsmittels mit Verbindungen der Formel VI umgesetzt wird, oder daß
c) eine Verbindung der Formel V in beliebiger Reihenfolge und unter Anwendung eines Kondensationsmittels mit Verbindungen der Formel VI und der Formel VII umgesetzt wird, 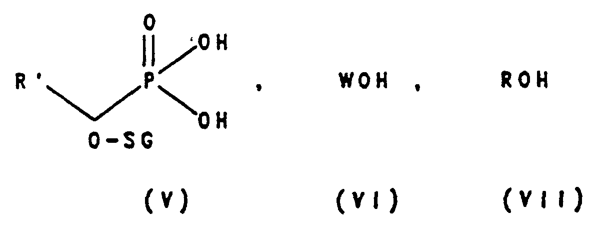
wobei SG eine Schutzgruppe darstellt, die zur Gewinnung der Verbindung der Formel I ggf. abgespalten wird,
oder eine Nucleotideinheit mit 3'(2')-terminaler H-Phosphonat Gruppierung und geschützter 5'-Hydroxygruppe mit einer weiteren Nucleotideinheit mit freier 5'-Hydroxygruppe und geschützter 3'(2')-Hydroxygruppe in Gegenwart eines Aktivierungsmittels zum H-Phosphosphonatdinucleosid umgesetzt und dieses mit einem Aldehyd zum Dinucleosid-α-hydroxyalkyl(aryl)-phosphonat kondensiert wird, das nach Umsetzung zu deren aktivierten Derivaten mit weiteren (Oligo)-Nucleotidfragmenten zu Oligonucleotiden reagiert, wobei temporär eingeführte Schutzgruppen abespalten werden, oder daß
a) eine Nucleotideinheit mit 3'(2')-terminaler Phosphor(III) oder Phosphor(V)-Gruppierung mit einer freien 5'-Hydroxygruppe einer weiteren Nucleotideinheit bzw. wachsenden Oligonucleotidkette in Gegenwart eines Kondensationsmittels oder
b) deren aktivierten Derivaten umgesetzt wird, oder das Oligonucleotidanalogen in Fragmenten in gleicher Weise aufgebaut wird, in den nach a) oder b) erhaltenen Oligonucleotiden zum Schutz anderer Funktionen temoprär eingeführte Schutzgruppen abgespalten werden und die so erhaltenen Oligonucleotid-Analoga der Formel I worin W einen Rest der Formel II und R einen Rest der Formel II' bedeuten gegebenenfalls in ihr physiologisch verträgliches Salz überführt werden.

7. Verwendung einer verbindung der Formel I gemäß einem oder mehreren der Ansprüche 1-5 zur Herstellung eines Arzneimittels.

8. Arzneimittel, enthaltend eine oder mehrere der Verbindungen der Formel I gemäß einem oder mehreren der Ansprüche 1-5-

9. Verbindungen der Formel I gemäß einem oder mehreren der Ansprüche 1-5 zur Anwendung als Arzneimittel.

## Claims

1. Compounds of the formula I, 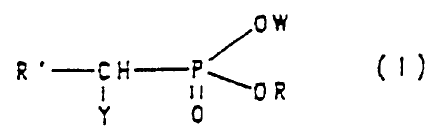 **characterized in that**
Y has the meaning of OH, SH, OAc or SAc, where Ac=(C1-C8)-acyl, which is optionally unsaturated 1-3 times,
R' is aryl having 6-14 carbon atoms, optionally substituted by up to three radicals which are independent of one another, selected from the group consisting of
(C1-C5)-alkyl, halogen, NO₂, CN, (C1-C6)-alkoxy, amino, (C1-C4)-alkylamino, (C1-C8)-dialkylamino, where a (C3-C8)-alkylene radical in which a CH₂ group can also be replaced by oxy can also be fused onto the aryl radical;
heteroaryl having 3 to 13 carbon atoms and up to 3 heteroatoms selected from the group consisting of N, O and S;
(C1-C16)-alkyl, which is branched or unbranched, saturated or unsaturated 1-3 times, optionally substituted independently of one another by up to three substituents selected from the group consisting of halogen, CN, NO₂ and (C1-C3)-alkoxy,
W has the meaning of a pharmaceutically active 5', 3' or 2' nucleoside analogue,
R has the meaning of W, where R and W can be identical or different, or R is (C1-C16)-alkyl, which can be branched or unbranched and is optionally substituted independently of one another by up to 3 radicals from the group consisting of halogen, CN, (C1-C8)-acyloxy, (C1-C18)-alkoxy or
W and R, together with the phosphonate radical carrying them, form an oligonucleotide where W is a radical of the formula II or a modified oligonucleotide and R is a radical of the formula II' or a modified oligonucleotide 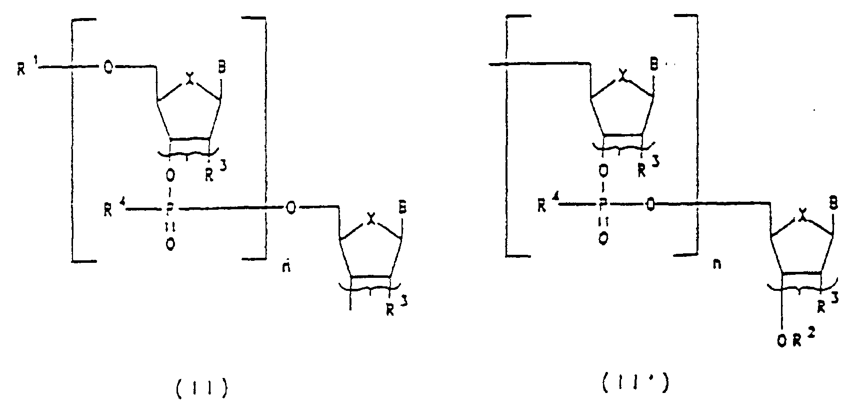
where X is oxy sulphanediyl,
B independently of one another is a nucleotide base,
n independently of one another is an integer from 0 to 30,
R¹ and R² independently of one another are H (C1-C12)acyl or a radical of the formula 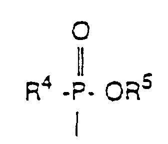
in which R⁴ is O, S, CH₃ or CHYR', where R' and Y are as defined above and R⁵ is an optionally substituted alkyl radical having 1-12 carbon atoms,
R³ is independently of one another H, O(C1-C12)-alkyl, O(C1-C12)-acyl, Cl, N₃, NH₂ or NHR⁶ where R⁶ is (C1-C3)-alkyl or -acyl
and the curved bracket indicates that R³ and the adjacent phosphonyl radical can be in the 2' or 3' position.

2. Compounds of the formula I according to Claim 1,
**characterized in that**
Y has the meaning of OH, SH, OAc or SAc, where Ac = (C1-C8)-acyl, which is optionally unsaturated 1-3 times,
R' is aryl having 6-14 carbon atoms, optionally substituted by up to three radicals which are independent of one another, selected from the group consisting of
(C1-C5)-alkyl, halogen, NO₂, CN, (C1-C6)-alkoxy, amino, (C1-C4)-alkylamino, (C1-C8)-dialkylamino, where a (C3-C8)-alkylene radical in which a CH₂ group can also be replaced by oxy can also be fused onto the aryl radical;
heteroaryl having 3 to 13 carbon atoms and up to 3 heteroatoms selected from the group consisting of N, O and S;
(C1-C16)-alkyl, which is branched or unbranched, saturated or unsaturated 1-3 times, optionally substituted independently of one another by up to three substituents selected from the group consisting of halogen, CN, NO₂ and (C1-C3)-alkoxy,
W has the meaning of a pharmaceutically active 5', 3' or 2' nucleoside analogue,
R has the meaning of W, where R and W can be identical or different, or R is (C1-C16)-alkyl, which can be branched or unbranched and is optionally substituted independently of one another by up to 3 radicals from the group consisting of halogen, CN, (C1-C8)-acyloxy, (C1-C18)-alkoxy or
W and R, together with the phosphonate radical carrying them, form an oligonucleotide where W is a radical of the formula II or a modified oligonucleotide and R is a radical of the formula II' or a modified oligonucleotide
where X is oxy or sulphanediyl,
B independently of one another is a nucleotide base,
n independently of one another is an integer from 0 to 30,
R¹ and R² independently of one another are H (C1-C12)-acyl or a radical of the formula 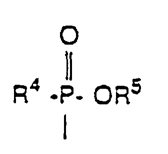
in which R⁴ is O, S, CH₃ or CHYR', where R' and Y are as defined above and R⁵ is an optionally substituted alkyl radical having 1-12 carbon atoms,
R³ independently of one another is H, O(C1-C12)-alkyl, O(C1-C12)-acyl, Cl, N₃, NH₂ or NHR⁶ where R⁶ is (C1-C3)-alkyl or -acyl
and the curved bracket indicates that R³ and the adjacent phosphonyl radical can be in the 2' or 3' position.

3. Compounds of the formula I according to Claims 1 or 2,
**characterized in that**
Y has the meaning of OH, SH, OAc or SAc, where Ac = (C1-C3)-acyl, which is optionally unsaturated 1-3 times,
R' is aryl having 6-14 carbon atoms, optionally substituted by up to 3 radicals which are independent of one another, selected from the group consisting of
(C1-C3)-alkyl, F, Cl, NO₂, CN, (C1-C4)-alkoxy, amino, (C1-C3)-alkylamino, (C1-C6)-dialkylamino, where a (C3-C8)-alkylene radical in which a CH₂ group can also be replaced by oxy can also be fused onto the aryl radical;
heteroaryl having 3 to 6 carbon atoms and up to 3 heteroatoms selected from the group consisting of N, O and S;
(C1-C8)-alkyl, which is branched or unbranched, saturated or unsaturated 1-3 times, optionally substituted independently of one another by up to three substituents selected from the group consisting of Cl, CN, NO₂ and (C1-C3)-alkoxy,
W has the meaning of a pharmaceutically active 5', 3' or 2' nucleoside analogue,
R has the meaning of W or is (C1-C8)-alkyl, which can be branched or unbranched and is optionally substituted by up to 2 radicals from the group consisting of halogen, CN, (C3-C6)-acyloxy, (C8-C18)-alkoxy or
W and R, together with the phosphonate radical carrying them, form an oligonucleotide where W is a radical of the formula II or a modified oligonucleotide and R is a radical of the formula II' or a modified oligonucleotide
where X is oxy,
B independently of one another is a nucleotide base,
n independently of one another is an integer from 0 to 20,
R¹ and R² independently of one another are H (C1-C8)-acyl or a radical of the formula 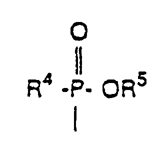
in which R⁴ is O, S, CH₃ or CHYR', where R' and Y are as defined above and R⁵ is an optionally substituted alkyl radical having 1-8 carbon atoms,
R³ independently of one another is H, O(C1-C8)-alkyl, O(C1-C8)-acyl, Cl or N₃, and the curved bracket indicates that R³ and the adjacent phosphonyl radical can be in the 2' or 3' position.

4. Compounds of the formula I according to one or more of Claims 1-3,
**characterized in that**
Y has the meaning of OH,
R' is aryl having 6 carbon atoms, optionally substituted by up to 3 radicals which are independent of one another, selected from the group consisting of
(C1-C3)-alkyl, F, Cl, NO₂, CN, (C1-C4)-alkoxy, amino, (C1-C3)-alkylamino, (C1-C6)-dialkylamino, where a (C3-C6)-alkylene radical in which a CH₂ group can also be replaced by oxy can also be fused onto the aryl radical;
heteroaryl having 3 to 6 carbon atoms and up to 3 heteroatoms selected from the group consisting of N, O and S;
(C1-C8)-alkyl, which is branched or unbranched, saturated or unsaturated 1-3 times, preferably unsaturated in conjugated form having an unsaturated bond in the alpha-position, optionally substituted independently of one another by up to three substituents selected from the group consisting of Cl, CN, NO₂ and (C1-C3)-alkoxy,
W has the meaning of a pharmaceutically active 5' or 3' nucleoside analogue,
R has the meaning of W or is (C1-C4)-alkyl, which can be branched or unbranched or
W and R, together with the phosphonate radical carrying them, form an oligonucleotide where W is a radical of the formula II or a modified oligonucleotide and R is a radical of the formula II' or a modified oligonucleotide
where X is oxy,
B independently of one another is a nucleotide base,
n independently of one another is an integer from 0 to 15,
R¹ and R² independently of one another are H (C1-C4)-acyl or a radical of the formula 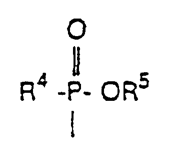
in which R⁴ is 0, S, CH₂ or CHYR', where R' and Y are as defined above and R⁵ is an optionally substituted alkyl radical having 1-3 carbon atoms,
R³ independently of one another is H, O(C1-C3)-alkyl, O(C1-C3)-acyl, Cl or N₃, and the curved bracket indicates that R³ and the adjacent phosphonyl radical can be in the 2' or 3' position.

5. Compounds of the formula I according to one or more of Claims 1-4,
**characterized in that**
Y has the meaning of OH,
W has the meaning of a 5'- or 3' nucleoside analogue,
R has the meaning of W or is (C1-C4)-alkyl, which can be branched or unbranched,
R' is aryl having 6 carbon atoms, optionally substituted by up to 3 radicals which are independent of one another, selected from the group consisting of
Cl, NO₂, CN, (C1-C3)-alkoxy, amino and (C1-C3)-alkylamino.

6. Process for the preparation of compounds according to one or more of Claims 1-5, **characterized in that**
a) a compound of the formula III is reacted with a compound of the formula IV, 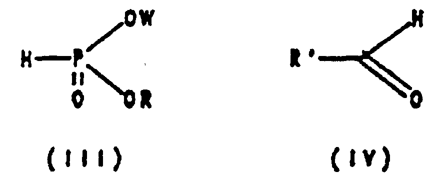 or in that
b) a compound of the formula V is reacted with compounds of the formula VI in any desired sequence and using a condensing agent,
or in that
c) a compound of the formula V is reacted with compounds of the formula VI and of the formula VII in any desired sequence and using a condensing agent, 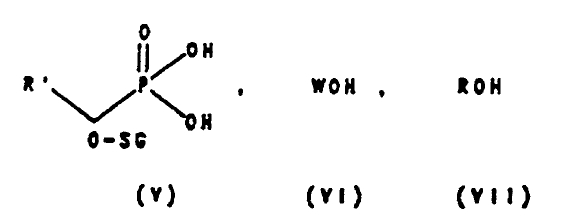
where SG is a protective group which is optionally removed to obtain the compound of the formula I,
or a nucleotide unit having a 3'(2')-terminal H-phosphonate group and protected 5'-hydroxy group is reacted with a further nucleotide unit having a free 5'-hydroxy group and protected 3'(2')-hydroxy group in the presence of an activating agent to give the H-phosphosphonate dinucleoside and this is condensed with an aldehyde to give the dinucleoside a-hydroxyalkyl(aryl)phosphonate, which after reaction to give its activated derivatives reacts with further (oligo)nucleotide fragments to give oligonucleotides, temporarily introduced protective groups being removed, or in that
a) a nucleotide unit having a 3'(2')-terminal phosphorus(III) or phosphorus(V) group is reacted with a free 5'-hydroxy group of a further nucleotide unit or growing oligonucleotide chain in the presence of a condensing agent or
b) its activated derivatives, or the oligonucleotide analogue is built up in fragments in the same way, protective groups temporarily introduced into the oligonucleotides obtained according to a) or b) for the protection of other functions are removed and the oligonucleotide analogues of the formula I thus obtained in which W is a radical of the formula II and R is a radical of the formula II' are optionally converted into their physiologically tolerable salt.

7. Use of a compound of the formula I according to one or more of Claims 1-5 for the production of a pharmaceutical.

8. Pharmaceuticals, containing one or more of the compounds of the formula I according to one or more of Claims 1-5.

9. Compounds of the formula I according to one or more of Claims 1-5 for use as pharmaceuticals.

## Revendications

1. Composés de formule I 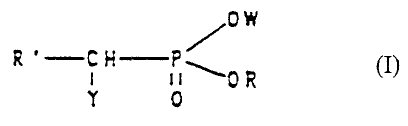 **caractérisés en ce que**
Y représente un reste OH, SH, OAc ou SAc, où Ac est un reste acyle en C₁-C₈ contenant éventuellement 1 à 3 insaturations,
R' représente un reste aryle de 6 à 14 atomes de carbone, éventuellement substitué par 1 à 3 restes indépendants les uns des autres et choisis dans le groupe constitué par les restes alkyle en C₁-C₅, halogéno, NO₂, CN, alcoxy en C₁-C₆, amino, alkylamino en C₁-C₄, dialkylamino en C₁-C₈, le reste aryle pouvant aussi être condensé avec un reste alkylène en C₃-C₈ dans lequel un groupe CH₂ peut être remplacé par un oxygène;
un reste hétéroaryle de 3 à 13 atomes de carbone et ayant jusqu'à 3 hétéroatomes choisis dans le groupe constitué par N, O et S;
un reste alkyle en C₁-C₁₆, linéaire ou ramifié, saturé ou contenant 1 à 3 insaturations, éventuellement substitué par 1 à 3 substituants choisis indépendamment les uns des autres dans le groupe constitué par les restes halogéno, CN, NO₂ et alcoxy en C₁-C₃,
W représente un analogue de 5', 3' ou 2'-nucléoside à activité pharmaceutique,
R a la signification de W, R et W pouvant être identiques ou différents, ou R représente un reste alkyle en C₁-C₁₆ linéaire ou ramifié et éventuellement substitué par 1 à 3 restes choisis indépendamment les uns des autres dans le groupe constitué par les restes halogéno, CN, acyloxy en C₁-C₈ et alcoxy en C₁-C₁₈, ou
W et R forment ensemble, avec le reste de phosphonate qui les porte, un oligonucléotide dans lequel W est un reste de formule II ou un oligonucléotide modifié et R est un reste de formule II' ou un oligonucléotide modifié 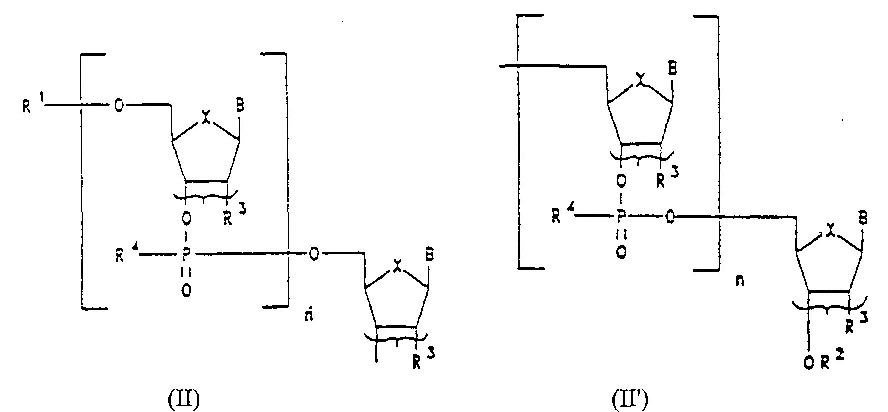 où X représente un reste oxy ou sulfanediyle,
les B représentent indépendamment les uns des autres une base nucléotidique,
les n représentent indépendamment les uns des autres un nombre entier de 0 à 30,
R¹ et R² représentent indépendamment l'un de l'autre H, un reste acyle en C₁-C₁₂ ou un reste de formule 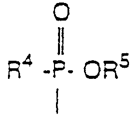 dans laquelle
R⁴ représente O, S, CH₃ ou CHYR', R' et Y ayant la signification donnée ci-dessus, et
R⁵ est un reste alkyle de 1 à 12 atomes de carbone éventuellement substitué,
les R³ représentent indépendamment les uns des autres H ou un reste O(alkyle en C₁-C₁₂), O(acyle en C₁-C₁₂), Cl, N₃, NH₂ ou NHR⁶, R⁶ étant un reste alkyle en C₁-C₃ ou acyle en C₁-C₃,
et l'accolade indique que R³ et le reste phosphonyle adjacent peuvent se trouver en position 2' ou 3'.

2. Composés de formule I selon la revendication 1, **caractérisés en ce que**
Y représente un reste OH, SH, OAc ou SAc, où Ac est un reste acyle en C₁-C₈ contenant éventuellement 1 à 3 insaturations,
R' représente un reste aryle de 6 à 14 atomes de carbone, éventuellement substitué par 1 à 3 restes indépendants les uns des autres et choisis dans le groupe constitué par les restes alkyle en C₁-C₅, halogéno, NO₂, CN, alcoxy en C₁-C₆, amino, alkylamino en C₁-C₄, dialkylamino en C₁-C₈, le reste aryle pouvant aussi être condensé avec un reste alkylène en C₃-C₈ dans lequel un groupe CH₂ peut être remplacé par un oxygène;
un reste hétéroaryle de 3 à 13 atomes de carbone et ayant jusqu'à 3 hétéroatomes choisis dans le groupe constitué par N, O et S;
un reste alkyle en C₁-C₁₆, linéaire ou ramifié, saturé ou contenant 1 à 3 insaturations, éventuellement substitué par 1 à 3 substituants choisis indépendamment les uns des autres dans le groupe constitué par les restes halogéno, CN, NO₂ et alcoxy en C₁-C₃,
W représente un analogue de 5', 3' ou 2'-nucléoside à activité pharmaceutique,
R a la signification de W, R et W pouvant être identiques ou différents, ou R représente un reste alkyle en C₁-C₁₆ linéaire ou ramifié et éventuellement substitué par 1 à 3 restes choisis indépendamment les uns des autres dans le groupe constitué par les restes halogéno, CN, acyloxy en C₁-C₈ et alcoxy en C₁-C₁₈, ou
W et R forment ensemble, avec le reste de phosphonate qui les porte, un oligonucléotide dans lequel W est un reste de formule II ou un oligonucléotide modifié et R est un reste de formule II' ou un oligonucléotide modifié, où X représente un reste oxy ou sulfanediyle,
les B représentent indépendamment les uns des autres une base nucléotidique,
les n représentent indépendamment les uns des autres un nombre entier de 0 à 30,
R¹ et R² représentent indépendamment l'un de l'autre H, un reste acyle en C₁-C₁₂ ou un reste de formule 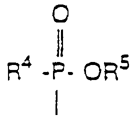 dans laquelle
R⁴ représente O, S, CH₃ ou CHYR', R' et Y ayant la signification donnée ci-dessus, et
R⁵ est un reste alkyle de 1 à 12 atomes de carbone éventuellement substitué, les R³ représentent indépendamment les uns des autres H ou un reste O(alkyle en C₁-C₁₂), O(acyle en C₁-C₁₂), Cl, N₃, NH₂ ou NHR⁶, R⁶ étant un reste alkyle en C₁-C₃ ou acyle en C₁-C₃,
et l'accolade indique que R³ et le reste phosphonyle adjacent peuvent se trouver en position 2' ou

3. Composés de formule I selon les revendications 1 ou 2, **caractérisés en ce que**
Y représente un reste OH, SH, OAc ou SAc, où Ac est un reste acyle en C₁-C₃ contenant éventuellement 1 à 3 insaturations,
R' représente un reste aryle de 6 à 14 atomes de carbone, éventuellement substitué par 1 à 3 restes indépendants les uns des autres et choisis dans le groupe constitué par les restes alkyle en C₁-C₃, F, Cl, NO₂, CN, alcoxy en C₁-C₄, amino, alkylamino en C₁-C₃, dialkylamino en C₁-C₆, le reste aryle pouvant aussi être condensé avec un reste alkylène en C₃-C₈ dans lequel un groupe CH₂ peut être remplacé par un oxygène;
un reste hétéroaryle de 3 à 6 atomes de carbone et ayant jusqu'à 3 hétéroatomes choisis dans le groupe constitué par N, O et S;
un reste alkyle en C₁-C₈, linéaire ou ramifié, saturé ou contenant 1 à 3 insaturations, éventuellement substitué par 1 à 3 substituants choisis indépendamment les uns des autres dans le groupe constitué par les restes Cl, CN, NO₂ et alcoxy en C₁-C₃,
W représente un analogue de 5', 3' ou 2'-nucléoside à activité pharmaceutique,
R a la signification de W ou représente un reste alkyle en C₁-C₈ linéaire ou ramifié et éventuellement substitué par 1 à 2 restes choisis dans le groupe constitué par les restes halogéno, CN, acyloxy en C₃-C₆ et alcoxy en C₈-C₁₈, ou
W et R forment ensemble, avec le reste de phosphonate qui les porte, un oligonucléotide dans lequel W est un reste de formule II ou un oligonucléotide modifié et R est un reste de formule II' ou un oligonucléotide modifié,
où X représente un reste oxy,
les B représentent indépendamment les uns des autres une base nucléotidique,
les n représentent indépendamment les uns des autres un nombre entier de 0 à 20,
R¹ et R² représentent indépendamment l'un de l'autre H, un reste acyle en C₁-C₈ ou un reste de formule 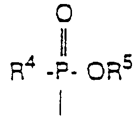 dans laquelle
R⁴ représente O, S, CH₃ ou CHYR', R' et Y ayant la signification donnée ci-dessus, et
R⁵ est un reste alkyle de 1 à 8 atomes de carbone éventuellement substitué,
les R³ représentent indépendamment les uns des autres H ou un reste O(alkyle en C₁-C₈), O(acyle en C₁-C₈), Cl ou N₃,
et l'accolade indique que R³ et le reste phosphonyle adjacent peuvent se trouver en position 2' ou 3'.

4. Composés de formule I selon l'une ou plusieurs des revendications 1 à 3, **caractérisés en ce que**
Y représente un reste OH,
R' représente un reste aryle de 6 atomes de carbone, éventuellement substitué par 1 à 3 restes indépendants les uns des autres et choisis dans le groupe constitué par les restes alkyle en C₁-C₃, F, Cl, NO₂, CN, alcoxy en C₁-C₄, amino, alkylamino en C₁-C₃, dialkylamino en C₁-C₆, le reste aryle pouvant aussi être condensé avec un reste alkylène en C₃-C₆ dans lequel un groupe CH₂ peut être remplacé par un oxygène;
un reste hétéroaryle de 3 à 6 atomes de carbone et ayant jusqu'à 3 hétéroatomes choisis dans le groupe constitué par N, O et S;
un reste alkyle en C₁-C₈, linéaire ou ramifié, saturé ou contenant 1 à 3 insaturations, de préférence insaturé sous forme conjuguée avec une liaison insaturée en position α, éventuellement substitué par 1 à 3 substituants choisis indépendamment les uns des autres dans le groupe constitué par les restes Cl, CN, NO₂ et alcoxy en C₁-C₃,
W représente un analogue de 5' ou 3'-nucléoside à activité pharmaceutique,
R a la signification de W ou représente un reste alkyle en C₁-C₄ linéaire ou ramifié ou
W et R forment ensemble, avec le reste de phosphonate qui les porte, un oligonucléotide dans lequel W est un reste de formule II ou un oligonucléotide modifié et R est un reste de formule II' ou un oligonucléotide modifié,
où X représente un reste oxy,
les B représentent indépendamment les uns des autres une base nucléotidique,
les n représentent indépendamment les uns des autres un nombre entier de 0 à 15,
R¹ et R² représentent indépendamment l'un de l'autre H, un reste acyle en C₁-C₄ ou un reste de formule 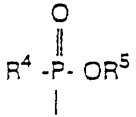 dans lequel
R⁴ représente O, S, CH₃ ou CHYR', R' et Y ayant la signification donnée ci-dessus, et
R⁵ est un reste alkyle de 1 à 3 atomes de carbone éventuellement substitué,
les R³ représentent indépendamment les uns des autres H ou un reste O(alkyle en C₁-C₃), O(acyle en C₁-C₃), Cl ou N₃,
et l'accolade indique que R³ et le reste phosphonyle adjacent peuvent se trouver en position 2' ou 3'.

5. Composés de formule I selon l'une ou plusieurs des revendications 1 à 4, **caractérisés en ce que**
Y représente un reste OH,
W représente un analogue de 5' ou 3'-nucléoside à activité pharmaceutique,
R a la signification de W ou représente un reste alkyle en C₁-C₄ linéaire ou ramifié,
R' représente un reste aryle de 6 atomes de carbone, éventuellement substitué par 1 à 3 restes indépendants les uns des autres et choisis dans le groupe constitué par les restes Cl, NO₂, CN, alcoxy en C₁-C₃, amino et alkylamino en C₁-C₃.

6. Procédé de préparation de composés selon l'une ou plusieurs des revendications 1 à 5, **caractérisé en ce que**
a) on fait réagir un composé de formule III avec un composé de formule IV 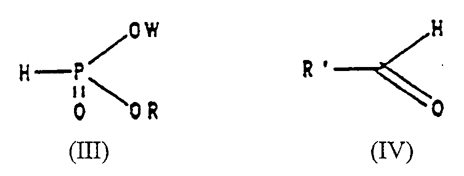 ou en ce que
b) on fait réagir un composé de formule V, dans un ordre quelconque et en présence d'un agent de condensation, avec des composés de formule VI, ou en ce que
c) on fait réagir un composé de formule V, dans un ordre quelconque et en présence d'un agent de condensation, avec des composés de formule VI et de formule VII 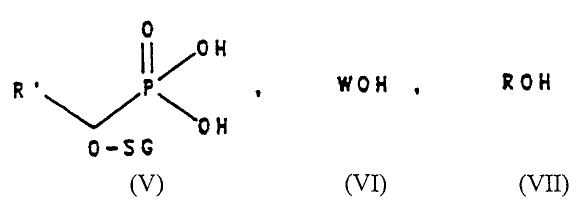 où SG représente un groupe protecteur que l'on sépare éventuellement pour obtenir le composé de formule I,
ou on fait réagir une unité de nucléotide ayant un groupement H-phosphonate terminal en 3'(2') et un groupe 5'-hydroxy protégé avec une autre unité de nucléotide ayant un groupe 5'-hydroxy libre et un groupe 3'(2')-hydroxy protégé en présence d'un agent d'activation pour obtenir le H-phosphonate-dinucléoside, et on condense ce dernier avec un aldéhyde pour former le dinucléoside-α-hydroxyalkyl(aryl)-phosphonate, que l'on fait réagir, après l'avoir transformé en son dérivé activé, avec d'autres fragments d'(oligo)nucléotides pour former des oligonucléotides, et on sépare les groupes protecteurs temporairement introduits; ou en ce que
a) on fait réagir une unité de nucléotide ayant un groupement à base de phosphore(III) ou de phosphore(V) terminal en 3'(2') avec un groupe 5-hydroxy libre d'une autre unité de nucléotide ou d'une chaîne oligonucléotidique croissante en présence d'un agent de condensation, ou avec
b) leurs dérivés activés, on synthétise l'analogue d'oligonucléotides en fragments de la même manière, on sépare les groupes protecteurs introduits temporairement pour la protection d'autres fonctions dans les oligonucléotides obtenus selon a) ou b) et on transforme éventuellement les analogues d'oligonucléotides de formule I ainsi obtenus, dans lesquels W est un reste de formule II et R un reste de formule II', en un sel physiologique acceptable.

7. Utilisation d'un composé de formule I selon l'une ou plusieurs des revendications 1 à 5 pour la préparation d'un médicament.

8. Médicament contenant un ou plusieurs composés de formule I selon l'une ou plusieurs des revendications 1 à 5.

9. Composés de formule I selon l'une ou plusieurs des revendications 1 à 5 à utiliser comme médicaments.
